# EUROPEAN PATENT SPECIFICATION

(11) **EP 2 552 924 B1**
(45) Date of publication and mention of the grant of the patent: **16.09.2015**
(21) Application number: 11715634.9
(22) Date of filing: 04.04.2011
(51) Int. Cl.: C07D 489/12, A61K 31/439, A61P 25/30

(54) **TRANSDERMALLY DELIVERABLE OPIOID PRODRUGS, ABUSE-RESISTANT COMPOSITIONS AND METHODS OF USING OPIOID PRODRUGS**
TRANSDERMAL FREISETZBARE OPIOID-PRODRUGS, MISSBRAUCHSRESISTENTE ZUSAMMENSETZUNGEN UND VERFAHREN ZUR VERWENDUNG VON OPIOID-PRODRUGS
PRODROGUES OPIOÏDES ADMINISTRABLES PAR VOIE TRANSDERMIQUE, COMPOSITIONS ANTI-ABUS ET MÉTHODES D'UTILISATION DE PRODROGUES OPIOÏDES

(30) Priority: 02.04.2010 US 320514 P; 02.04.2010 US 320522 P
(43) Date of publication of application: 06.02.2013
(73) Proprietor: Zynerba Pharmaceuticals, Inc., Radnor, PA 19087 (US)
(72) Inventor: STINCHCOMB, Audra, Lynn, Lexington KY 40505 (US); LI, Guohua, Lexington KY 45013 (US); BANKS, Stan, Lee, Frankfort KY 40601 (US); HOWARD, Jeffery, Lynn, Richmond KY 40475 (US); GOLINSKI, Miroslaw, Jerzy, Lexington KY 40514 (US)
(74) Representative: Grund, Martin
(86) International application number: PCT/US2011/031130
(87) International publication number: WO 2011/123863

(56) References cited:
- WO-A1-2008/036980
- FASSIHI ET AL.: "Racemates and enantiomers in drug development", INTERNATIONAL JOURNAL OF PHARMACEUTICS, vol. 92, no. 1-3, 3 May 1993 (1993-05-03), pages 1-14, XP025557939, ISSN: 0378-5173, DOI: 10.1016/0378-5173(93)90257-G [retrieved on 1993-05-03]
- KHAN ET AL.: "Single enantiomer drugs: should they be developed?", ESSENTIAL PSYCHOPHARMACOLOGY, vol. 7, no. 1, 1 January 2006 (2006-01-01) , pages 15-23, XP008127389, ISSN: 1087-495X
- AGRANAT ET AL.: "The strategy of enantiomer patents of drugs", DRUG DISCOVERY TODAY, vol. 15, no. 5-6, 1 March 2010 (2010-03-01), pages 163-170, XP026946346, ISSN: 1359-6446, DOI: 10.1016/J.DRUDIS.2010.01.007 [retrieved on 2010-01-29]

## Description

This application claims the benefit of U.S. Provisional Application Ser. No. 61/320,514, filed April 2, 2010 and U.S. Provisional Application Ser. No. 61/320,522, filed April 2,2010.

### FIELD

Described herein are opioid agonist-antagonist prodrugs, compositions for transdermal delivery of opioid agonist-antagonist prodrugs, abuse-resistant formulations and dosage forms for transdermal delivery of the opioid agonist-antagonist prodrugs, and methods of using such compositions, formulations and dosage forms in treating and preventing diseases and disorders.

### BACKGROUND

Pain is the most frequently reported symptom and is a common clinical problem which confronts the clinician. Millions of people in the United States suffer from severe pain that, according to numerous recent reports, is chronically under-treated or inappropriately managed.

Opioids have long been recognized as one of the most effective treatments of pain. However, they also have a high potential of abuse. In fact, opioid and narcotic abuse are major worldwide problems connected with tremendous social and personal strife. As of 1992, the estimated United States economic cost of drug and alcohol abuse was $246 billion. The latest National Household Survey on Drug Abuse survey conducted by the Substance Abuse and Mental Health Services Administration reported in July 2007 that nearly one in twelve full-time workers in the United States have serious enough drug/alcohol problems to require medical treatment. Providing recovery assistance for drug addicts and alcoholics with pharmacological interventions has proven helpful.

Certain opioids, such as buprenorphine, butorphanol, dezocine, meptazinol, nalbuphine and pentazocine, have both agonist and antagonist qualities. For example, the main agonist-antagonist effect of buprenorphine is through its binding to µ-opioid and κ-opioid receptors, acting clinically as an agonist at lower doses and as an antagonist at higher doses. The dual agonist-antagonist activity of these opioids make them effective at not only treating pain, but also at reducing the severity of the withdrawal symptoms experienced when a former abuser begins to eliminate opioid and/or alcohol. Buprenorphine is currently available as a sublingual dosage form, both alone (Subutex®) and in combination with naloxone (Suboxone®) for the treatment of pain and opioid dependence. Because they are administered sublingually, both have clinically relevant drawbacks. For example, the necessity of taking multiple daily doses, or even once-daily dosing, decreases patient compliance. In addition, the daily and multiple daily dosing necessary with sublingual dosage forms may cause more frequent and more extreme peaks and troughs in the blood-plasma concentration of the active medications, thereby, increasing the potential for a patient to experience both the adverse effects associated with supra-therapeutic concentrations and ineffective relief associated with sub-therapeutic concentrations.

Further, lack of appetite, nausea and/or frequent emesis are commonly experienced by patients undergoing withdrawal from narcotic or alcohol abuse and those suffering from chronic, under-treated or intractable pain. As such, oral and sublingual therapies for these patients are often either poorly tolerated or fail to provide an effective therapeutic dose.

For these patients, transdermal administration can provide a favorable route of administration. Transdermal dosing provides the patient with a desirable systemic delivery profile which can minimize or eliminate any "highs" (dizziness and drowsiness) associated with more rapid absorption and can reduce the side effects associated with oral administration of a drug, such as abdominal pain, nausea and vomiting. Additionally, transdermal administration avoids first-pass metabolism which can allow for higher therapeutic concentrations to be achieved. Transdermal delivery also offers a patient freedom from injections and surgical implantations. Transdermal delivery can also improve patient compliance by reducing the dose frequency. A transdermal patch can offer sustained release of a drug for an extended period (e.g., one week) while transdermal gels are also an accepted dosage form for convenient daily application.

Because of the inherent potential for abuse, it is important that any pharmaceutical composition containing an opioid agonist or opioid agonist-antagonist or prodrugs of either be made as abuse-resistant or abuse-deterrent as possible. This is particularly true with extended release opioid products, including transdermal applications. Illicit users often will attempt to circumvent the extended release properties of these dosage forms by injecting or otherwise misusing or abusing the product in order to achieve an immediate release of the opioid agonist or opioid agonist-antagonist.

The Food and Drug Administration ("FDA") has recently emphasized the importance of reducing the risk of opioid abuse. In a February 9, 2009 press release, the FDA publicly announced a program in which it would meet with the manufacturers of extended release and transdermal opioids regarding opioid misuse and abuse. Under the terms of the announced program, the manufactures will be required to develop Risk Evaluation and Mitigations Strategies to ensure proper opioid use.

Not all opioids however are capable of dermal absorption. Buprenorphine, for example, has been evaluated for transdermal delivery, but has generally been found to be too hydrophobic to cross the skin at a therapeutic rate through a reasonably-sized transdermal patch. However, as transdermal pharmaceutical compositions pass through the epidermis and dermis of many mammals, such as humans and guinea pigs, they are exposed to enzymes which are capable of metabolizing active pharmaceutical agents.
WO2008036980 A1 describes prodrugs of the opioid buprenorphine, and also deals with their transdermal administration.
The metabolic processes occurring in the skin of mammals, such as humans, can be utilized to deliver pharmaceutically effective quantities of opioids to a mammal in need thereof, by metabolizing prodrugs into active pharmaceutical compounds. Thus, it would be desirable to deliver prodrugs of buprenorphine, which are more hydrophilic than buprenorphine, through the skin at a higher rate than buprenorphine while taking advantage of the rapid hydrolysis of the buprenorphine prodrug to buprenorphine during transport into and through the skin. It would be further desirable to combine the buprenorphine prodrug with a non-dermally absorbed opioid antagonist, such as naltrexone (NTX) or naloxone (NLX), or a prodrug of an opioid antagonist, such as a prodrug of naltrexone or naloxone, in order to increase the abuse deterrence of the composition.

### SUMMARY

The invention is defined by the appending claims.

Described herein are prodrugs of opioid agonist-antagonist, including buprenorphine, methods of making prodrugs of buprenorphine, compositions comprising prodrugs of buprenorphine, abuse-resistant formulation and dosage forms for transdermal delivery of prodrugs of buprenorphine and methods of using prodrugs of buprenorphine.

### BRIEF DESCRIPTION OF THE DRAWINGS

Figure 1 is a plot of the cumulative permeation profile of buprenorphine base and the summation of buprenorphine from Formula IV (ALL00160) and intact Formula IV(ALL00160) versus time.
Figure 2 is a plot of the representative permeation profile of 5% Formula IV (ALL00160) buprenorphine prodrug patch (n=4) and 7% Formula IV (ALL00160) buprenorphine prodrug patch (n=4) versus time.

### DESCRIPTION

The present invention is defined by the appending claims.

The description that follows is subject to this limitation. All aspects and embodiments which are not covered by the claims are merely aspects of the present disclosure and do not form part of the invention.

The term prodrug as used herein refers to a pharmacologically inactive (or significantly less active) chemical derivative that can be converted, enzymatically or non-enzymatically, *in vivo* or *in vitro,* to an active drug molecule, which is capable of exerting one or more physiological effects.

Compounds described herein include pharmaceutically acceptable prodrugs of buprenorphine. Further, compositions described herein comprise at least one pharmaceutically acceptable prodrug of buprenorphine. One embodiment described herein includes pharmaceutically acceptable prodrugs of buprenorphine which are more hydrophilic than buprenorphine and suitable for transdermal administration to a mammal, such as humans. The buprenorphine prodrugs described herein may be in any form suitable for administration to a mammal, such as in the form of a free base, free acid, salt, ester, hydrate, anhydrate, stereo isomer (enantiomer and diastereomer), isomer, tautomer, polymorph, derivative, or the like, provided that the free base, salt, ester, hydrate, enantiomer, isomer, tautomer, or any other pharmacologically suitable derivative is able to undergo conversion to a therapeutically active form of buprenorphine.

Compositions described herein also include those which are suitable for transdermal administration of prodrugs of buprenorphine and, optionally, include a vehicle or carrier for the transdermal administration of a prodrug of buprenorphine (e.g., a transdermal patch), as well as, further comprising one or more of the following: pharmacologically active agents, solvents, thickening agents, penetration enhancers, wetting agents, lubricants, emollients, substances added to mask or counteract a disagreeable odor, fragrances, and substances added to improve appearance or texture of the composition as well as other excipients.

Methods of treating one or more medical conditions such as opioid dependence, alcohol dependence, polydrug addiction, pain, cocaine addiction, eating disorders (e.g., binge eating) and treatment-resistant depression are described herein and comprise transdermally administering a pharmaceutically acceptable prodrug of buprenorphine to a mammal, such as humans.

"Pharmaceutically acceptable salts," or "salts," include the salts of buprenorphine prodrugs, suitable for administration to a mammal, and includes those prepared from formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic, methanesulfonic, ethanesulfonic, benzenesulfonic, pantothenic, toluenesulfonic, 2-hydroxyethanesulfonic, sulfanilic, cyclohexylaminosulfonic, beta-hydroxybutyric, galactaric and galacturonic acids. The following list of pharmaceutically acceptable salts is not meant to be exhaustive but merely illustrative as a person of ordinary skill in the art would appreciate that other pharmaceutically acceptable salts of buprenorphine and buprenorphine prodrugs may be prepared.

In one embodiment, acid addition salts can be prepared from the free base forms through a reaction of the free base with a suitable acid. Suitable acids for preparing acid addition salts include, but are not limited to, both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid, phosphoric acid, and the like. The following list of organic and inorganic acids is not meant to be exhaustive but merely illustrative as a person of ordinary skill in the art would appreciate that other acids may be used to create pharmaceutically acceptable salts of buprenorphine and prodrugs of buprenorphine. In other embodiments, an acid addition salt is reconverted to the free base by treatment with a suitable base. In still other embodiments, the basic salts are alkali metal salts, e.g., sodium salt.

In one embodiment, illustrative opioid prodrugs include those compounds of Formula (I): 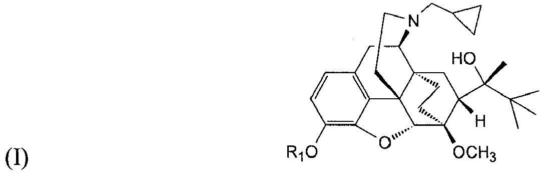 wherein R₁ is comprised of a bio-labile linker and further comprising moieties which can be selected in order to control the rate and extent of transdermal absorption and metabolism (e.g., increase hydrophilicity). Several options for R₁ are disclosed herein so that the resulting prodrug of buprenorphine is more hydrophilic than buprenorphine. Suitable options for R₁ include isomers of R₁, such as constitutional isomers, stereoisomers, enantiomers, diastereomers, and configurational diastereomers. Also included herein are pharmaceutically acceptable forms of Formula (I), including the free base, salt, ester, hydrate, polymorph and derivative of compounds of Formula I.

In additional embodiments of compounds of Formula (I), R₁ is an alkyl carbonate or an oxygenated alkyl carbonate prepared by functionalizing the 3-phenolic hydroxyl group present within the molecular structure of buprenorphine. In a further embodiment R₁ is a hydroxylated or non-hydroxylated alkyl carbonate. In another embodiment R₁ is an oxa-carbonate or a non-hydroxylated oxa-carbonate. In a further embodiment, R₁ is a pegylated carbonate which does not terminate in a methyl group and optionally does not terminate in a hydroxyl group. In a further embodiment, R₁ is a pegylated carbonate which terminates in an ethyl group. In another embodiment, R₁ is a pegylated carbonate having 1 ethylene glycol unit terminating in an ethyl group, 2 ethylene glycol repeat units terminating in an ethyl group, 3 ethylene glycol repeat units terminating in an ethyl group, 4 ethylene glycol repeat units terminating in an ethyl group, 5 ethylene glycol repeat units terminating in an ethyl group, 6 ethylene glycol repeat units terminating in an ethyl group, 7 ethylene glycol repeat units terminating in an ethyl group, 8 ethylene glycol repeat units terminating in an ethyl group, 9 ethylene glycol repeat units terminating in an ethyl group, 10 ethylene glycol repeat units terminating in an ethyl group, 11 ethylene glycol repeat units terminating in an ethyl group or 12 ethylene glycol repeat units terminating in an ethyl group.

In another embodiment the pegylated carbonate (i) optionally does not terminate with a hydroxyl group, (ii) optionally has between 1 and 12 ethylene glycol units, (iii) optionally does not terminate in a methyl group and (iv) is a buprenorphine prodrug which is more hydrophilic than buprenorphine. In another embodiment R₁ is a pegylated carbonate having between 1 and 12 ethylene glycol units and optionally terminates in an ethyl group and wherein the compound is more hydrophilic than buprenorphine. Additional embodiments include pharmaceutically acceptable salts of the prodrugs of buprenorphine described herein. Suitable salts can be found in Berge, 1977, "Pharmaceutical Salts," J. Pharm. Sci., 66:1-19.

| **Formula** | **Reference Name** | **Chemical Name** |
|---|---|---|
| I | ALL00157 | Buprenorphine 3,6-dioxaoctyl carbonate |
| II | ALL00158 | Buprenorphine 3,6,9,12-tetraoxatetradecanyl carbonate |
| III | ALL00159 | Buprenorphine 3,6,9-trioxaundecyl carbonate |
| IV | ALL00160 | Buprenorphine (3S)-3,4-dihydroxybutyl carbonate |
| V | ALL00160a | Buprenorphine (3R)-3,4-dihydroxybutyl carbonate |

In a further embodiment, one or more buprenorphine prodrug is selected from the group consisting of: 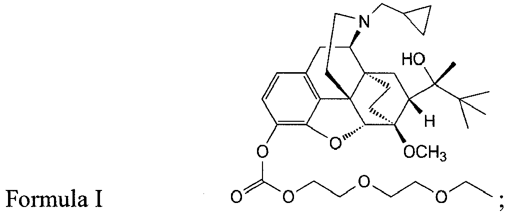 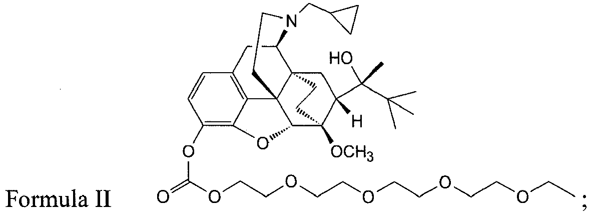 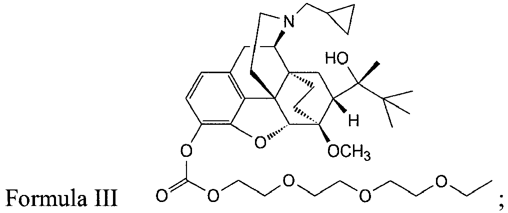 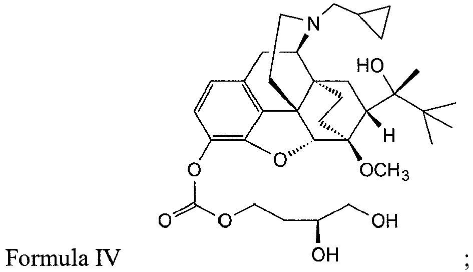 and 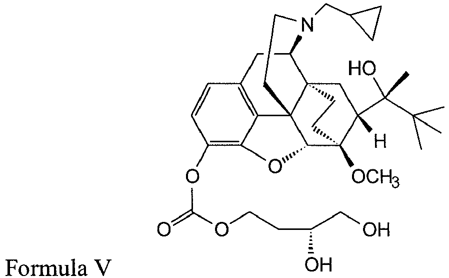 and salts of Formulas I-V.

Further embodiments include the 3S stereoisomer of Formula IV in substantially pure form, compositions (e.g., transdermal patches) comprising the 3S stereoisomer of Formula IV in substantially pure form and methods of using the 3S stereoisomer of Formula IV in substantially pure form.

Further embodiments include the 3R stereoisomer of Formula V in substantially pure form, compositions (e.g., transdermal patches) comprising the 3R stereoisomer of Formula V in substantially pure form and methods of using the 3R stereoisomer of Formula V in substantially pure form.

Further embodiments described herein are pharmaceutical compositions comprising:
(a) a buprenorphine prodrug or a salt thereof selected from the group consisting of: 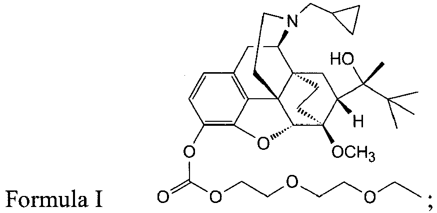 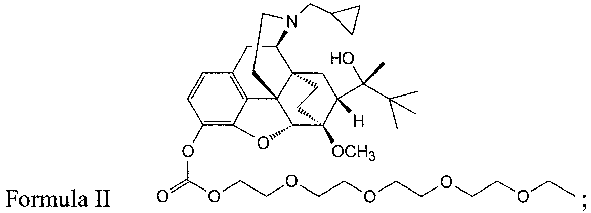 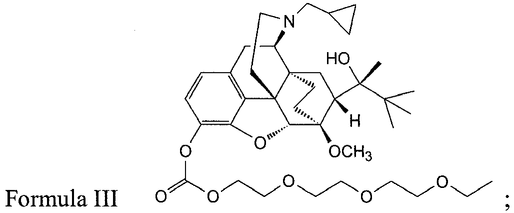 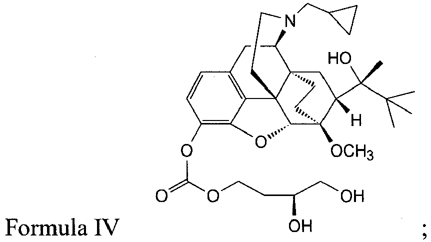 and 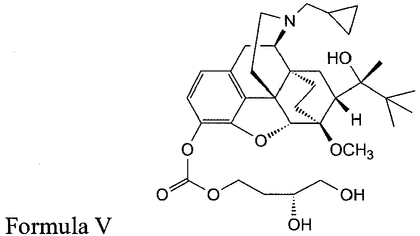 and
(b) a pharmaceutical excipient.

Disclosed is a method of applying a buprenorphine prodrug or a salt thereof to a mammal comprising the steps of:
(a) obtaining a pharmaceutical composition comprising:
   (i) a compound selected from the group consisting of: 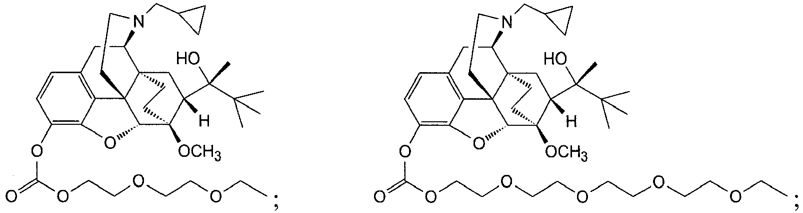 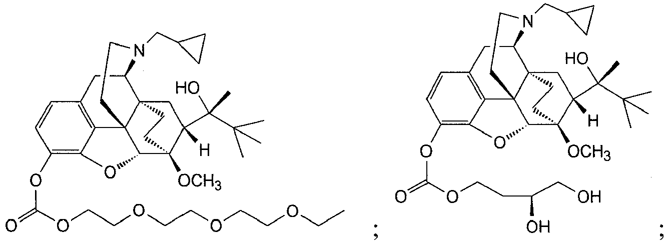 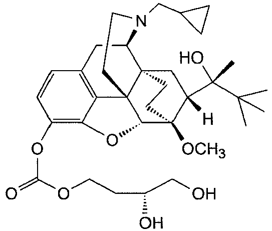 and salts of the foregoing; and
   (ii) a pharmaceutically acceptable excipient; and
(b) contacting the pharmaceutical composition with the skin of the mammal.

A further embodiment includes a buprenorphine prodrug having an *in vitro* transdermal flux enhancement of greater than one relative to buprenorphine.

A further embodiment includes a buprenorphine prodrug having an *in vitro* transdermal flux (nmol/cm²/hr) greater than buprenorphine.

A further embodiment includes a buprenorphine prodrug having a twenty-four hour cumulative amount (nmol) of *in vitro* transdermal permeation greater than buprenorphine.

Disclosed is a method of treating a medical condition in a mammal comprising the step of applying a buprenorphine prodrug or a salt thereof to the skin of a mammal from the group consisting of: 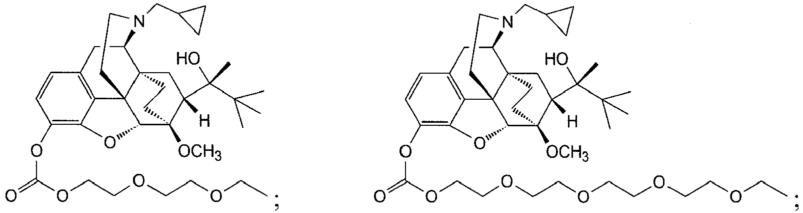 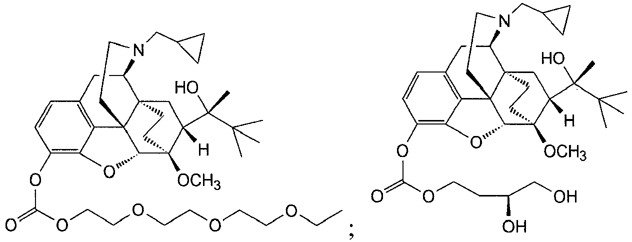 and 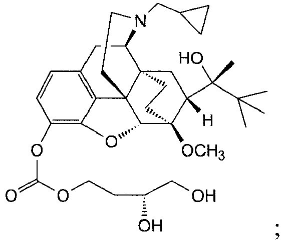 wherein the medical condition is selected from the group consisting of: opioid dependence, alcohol dependence, polydrug addiction, pain, cocaine addiction, eating disorders (e.g., binge eating) and treatment-resistant depression.

### Abuse-Resistant Compositions

Due to the potential for opioid agonists and opioid agonist-antagonists to be abused by individuals addicted to opioids, it is desirable to incorporate such compounds into abuse-resistant or abuse-deterrent formulations and dosage forms so that the possibility of abuse through intravenous administration, inhalation, oral ingestion or other methods is substantially reduced or eliminated. For example, with transdermal administration, it is desirable to use poorly absorbed forms of opioid antagonists to minimize the effect of the opioid antagonist during transdermal use, but preserving the antagonist properties in the event that abuse of the dosage form is attempted.

In one embodiment, the pharmaceutical composition contains an opioid agonist or agonist-antagonist, such as buprenorphine, or prodrugs of an opioid agonist or agonist-antagonist, such as a prodrug of buprenorphine, and an opioid antagonist or prodrug of an opioid antagonist. In a further embodiment, the opioid antagonist is selected from the group consisting of: naltrexone, 6-beta-naltrexol, nalmefene, naloxone and prodrugs of the foregoing.

In a further embodiment, illustrative opioid antagonist prodrugs include those compounds of Formula (X): 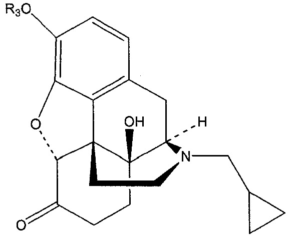 wherein R₃ is comprised of a bio-labile linker (e.g. ester, carbonate, carbamate, or other suitable bio-labile linking structure) and further comprising moieties which can be selected in order to control the rate and extent of transdermal absorption and metabolism. Several options for R₃ are disclosed herein. Also included herein is the free base, salt, ester, hydrate, amide, enantiomer, isomer, tautomer, polymorph, or derivative thereof of compounds of Formula (X)

In one embodiment, R₃ is selected from the group consisting of Formula (X), wherein R₃ is selected from the group consisting of:

Formula (XI): -COC(CH₃)₃;

Formula (XII): -COCH(CH₃)₂;

Formula (XIII): -COCH₂CH(CH₃)₂;

Formula (XIV): -COCH(CH₂CH₃)₂;

Formula (XV): -CON(CH₂CH₃)_{2;}

Formula (XVI): CON(CH(CH₃)₂)₂;

Formula (XVII): -COOCH(CH₃)₂;

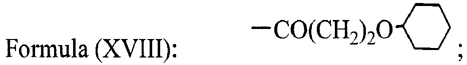

and

Formula (XIX): -CO(CH₂)₂OCH₃.

In one embodiment the opioid antagonist is selected from the group consisting of 3-O-pivalyl naltrexone, 3-O-isovaleryl naltrexone, 3-O-(2'-ethylbutyryl) naltrexone, 3-O-isobutyryl naltrexone, 3-O-isopropyloxycarbonyl naltrexone, 3-O-tertiarybutyloxycarbonyl naltrexone, N,N-dimethyl-3-O-carbamate naltrexone, N,N-diethyl-3-O-carbamate naltrexone, and N,N-diisopropyl-3-O-carbamate naltrexone. Other prodrugs of naltrexone, opioid antagonist prodrugs or opioid antagonists can also be used, such as naloxone and prodrugs of naloxone.

Further embodiments are pharmaceutical compositions comprising:
(a) a buprenorphine prodrug selected from the group consisting of: 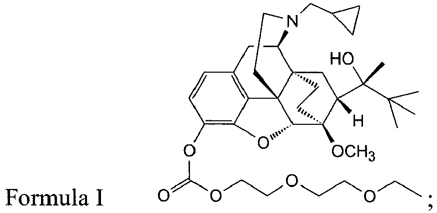 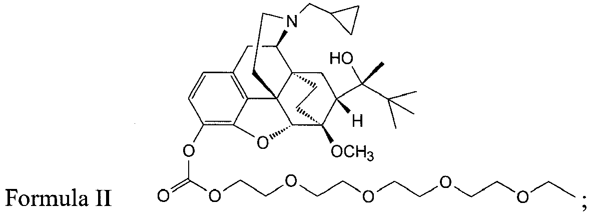 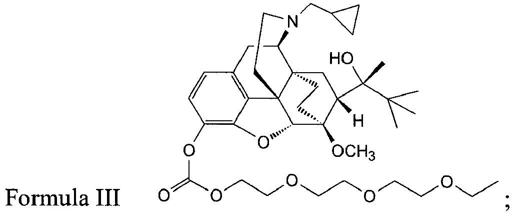 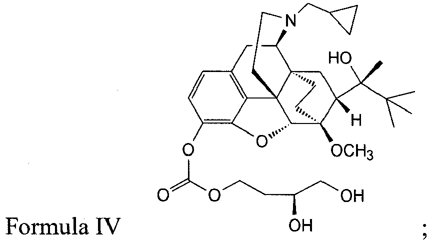 and 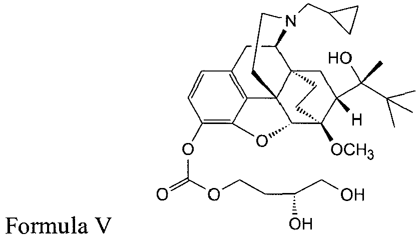
(b) a naltrexone prodrug of Formula (X), wherein R₃ is selected from:

   Formula (XI): -COC(CH₃)₃;

   Formula (XII): -COCH(CH₃)₂;

   Formula (XIII): -COCH₂CH(CH₃)₂;

   Formula (XIV): -COCH(CH₂CH₃)₂;

   Formula (XV): -CON(CH₂CH₃)_{2;}

   Formula (XVI): CON(CH(CH₃)₂)₂;

   Formula (XVII): -COOCH(CH₃)₂;

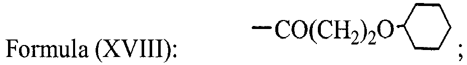

   and

   Formula (XIX): -CO(CH₂)₂OCH₃;

   and
(c) a pharmaceutical excipient.

Further embodiments are pharmaceutical compositions comprising:
(a) a buprenorphine prodrug selected from the group consisting of: 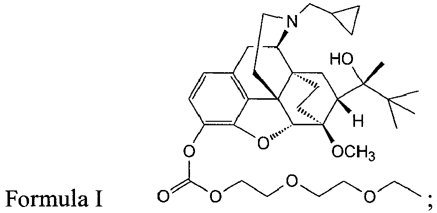 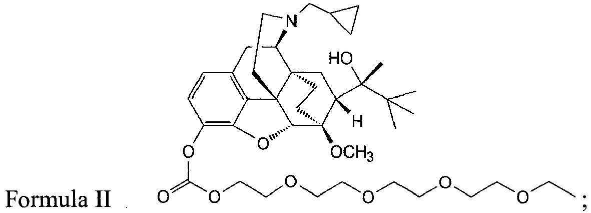 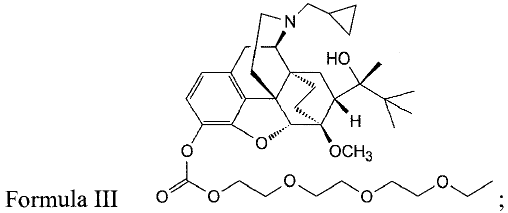 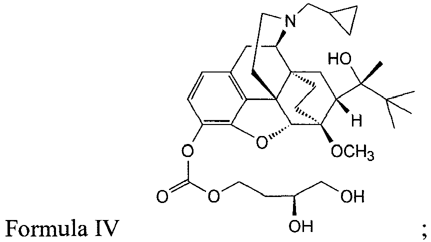 and 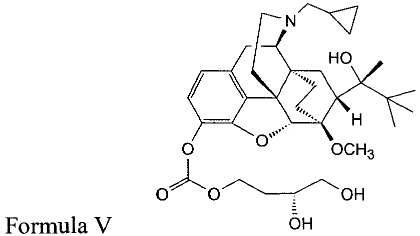
(b) naloxone or a naloxone prodrug; and
(c) a pharmaceutical excipient.

A further embodiment includes a method for transdermally delivering a buprenorphine prodrug or salt thereof to a mammal comprising the steps of:
(a) obtaining a pharmaceutical composition comprising:
   (i) a buprenorphine prodrug selected from the group consisting of: 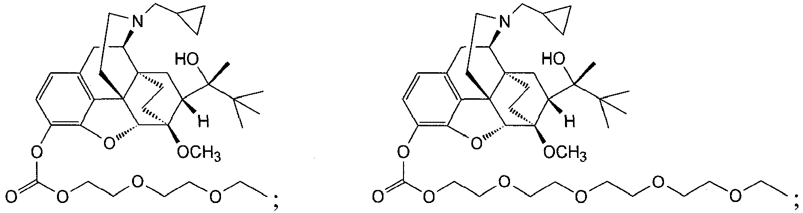 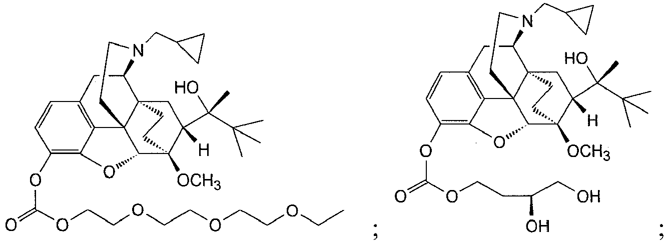 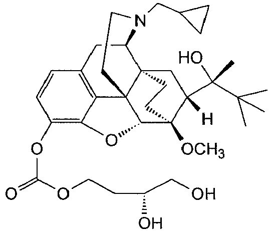 and salts of the foregoing; and
   (ii) a naltrexone prodrug of having the formula 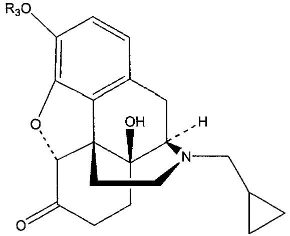 wherein R₃ is selected from the group consisting of: H; -COC(CH₃)₃; -COCH(CH₃)₂; -COCH₂CH(CH₃)₂; -COCH(CH₂CH₃)₂; -CON(CH₂CH₃)_{2;} -CON(CH(CH₃)₂)₂;-COOCH(CH₃)₂; 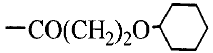 and -CO(CH₂)₂OCH₃; and
   (iii) a pharmaceutically acceptable excipient; and
(b) contacting the pharmaceutical composition with the skin of the mammal.

Further embodiments include methods of treating a medical condition in a mammal comprising the step of:
(a) obtaining a pharmaceutical composition comprising:
   (i) a buprenorphine prodrug or a salt thereof selected from the group consisting of: 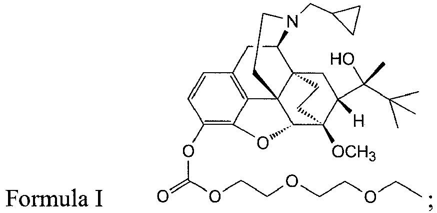 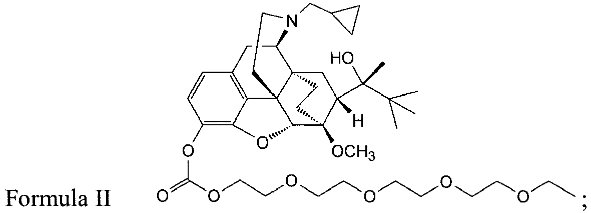 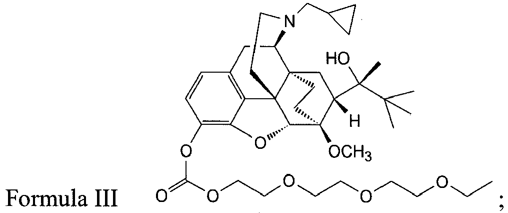 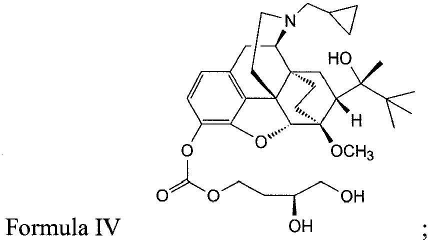 and 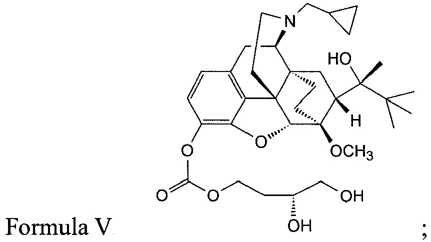 and
   (ii) a naltrexone prodrug or salt thereof of Formula (X), wherein R₃ is selected from the group consisting of:

      Formula (XI): -COC(CH₃)₃;

      Formula (XII): -COCH(CH₃)₂;

      Formula (XIII): -COCH₂CH(CH₃)₂;

      Formula (XIV): -COCH(CH₂CH₃)₂;

      Formula (XV): -CON(CH₂CH₃)_{2;}

      Formula (XVI): CON(CH(CH₃)₂)₂;

      Formula (XVII): -COOCH(CH₃)₂;

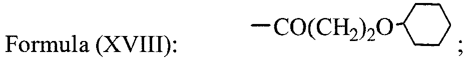

      and

      Formula (XIX): CO(CH₂)₂OCH₃;

      and
(b) contacting the pharmaceutical composition with the skin of a mammal.

Further embodiments include methods of treating a medical condition in a mammal comprising the step of:
(a) obtaining a pharmaceutical composition comprising:
   (i) a buprenorphine prodrug or a salt thereof selected from the group consisting of: 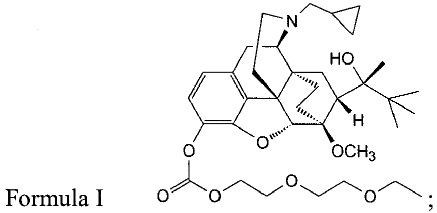 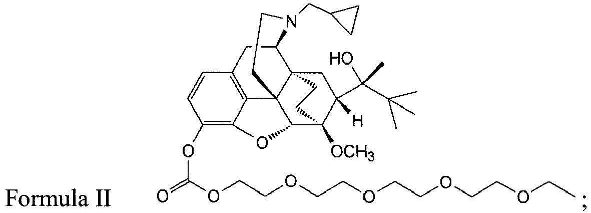 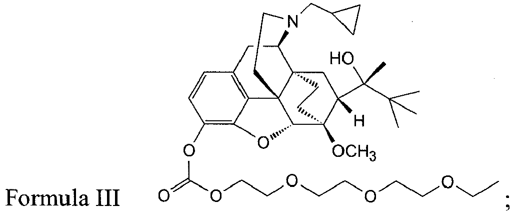 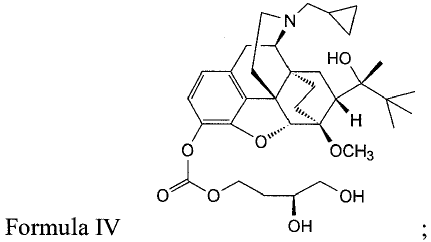 and 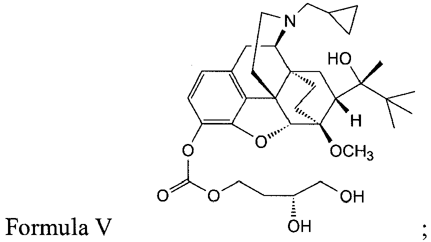 and
   (ii) naloxone or a prodrug of naloxone;
(b) contacting the pharmaceutical composition with the skin of a mammal.

In a further embodiment, the medical condition is selected from the group consisting of: opioid dependence, alcohol dependence, polydrug addiction, pain, cocaine addiction, eating disorders (e.g., binge eating) and treatment-resistant depression.

### Therapeutic Uses

Methods of treating one or more medical conditions, such as opioid dependence, alcohol dependence, polydrug addiction, pain, cocaine addiction, eating disorders (e.g., binge eating) and treatment-resistant depression, are described herein and comprise administering an opioid agonist or agonist-antagonist or prodrug of the foregoing in an abuse-resistant formulation. In one embodiment, compositions described herein include opioid agonists or agonist-antagonists, such as buprenorphine, and opioid antagonists, such as naltrexone and/or naloxone.

In another embodiment, compositions described herein include opioid agonists or agonist-antagonists, such as buprenorphine, and prodrugs of opioid antagonists, such as prodrugs of naltrexone and/or naloxone.

In another embodiment, compositions described herein include prodrugs of opioid agonists or agonist-antagonists, such as prodrugs of buprenorphine, and opioid antagonist prodrugs, such as prodrugs of naltrexone and/or naloxone.

In another embodiment, compositions described herein include prodrugs of opioid agonists or agonist-antagonists, such as prodrugs of buprenorphine, and opioid antagonist, such as naltrexone and/or naloxone.

In another embodiment, compositions disclosed herein comprise one or more opioid agonists or agonist-antagonists, including buprenorphine, in a total amount of about of between about 0.1% and about 95% by weight of the composition. For example, one or more opioid agonists or agonist-antagonists may be present in the amount by weight of: about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 4%, about 5%, about 6%, about 7%, about 8%, about 9%, about 10%, about 11%, about 12%, about 13%, about 14%, about 15%, about 20%, about 25%, about 30%, about 35%, about 40%, about 45%, about 50%, about 55%, about 60%, about 65%, about 70%, about 75%, about 80%, about 85%, about 90% or about 95%.

In another embodiment, compositions disclosed herein comprise one or more prodrugs of opioid agonists or agonist-antagonists, including prodrugs of buprenorphine, in a total amount of about of between about 0.1 % and about 95% by weight of the composition. For example, one or more opioid agonists or agonist-antagonists may be present in the amount by weight of: about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 4%, about 5%, about 6%, about 7%, about 8%, about 9%, about 10%, about 11%, about 12%, about 13%, about 14%, about 15%, about 20%, about 25%, about 30%, about 35%, about 40%, about 45%, about 50%, about 55%, about 60%, about 65%, about 70%, about 75%, about 80%, about 85%, about 90% or about 95%.

In another embodiment, compositions disclosed herein comprise one or more opioid antagonists, including naltrexone and/or naloxone, in a total amount of about of between about 0.1% and about 95% by weight of the composition. For example, one or more opioid antagonists maybe present in an amount by weight of: about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 4%, about 5%, about 6%, about 7%, about 8%, about 9%, about 10%, about 11%, about 12%, about 13%, about 14%, about 15%, about 20%, about 25%, about 30%, about 35%, about 40%, about 45%, about 50%, about 55%, about 60%, about 65%, about 70%, about 75%, about 80%, about 85%, about 90% or about 95%.

In another embodiment, compositions disclosed herein comprise one or more opioid antagonist prodrugs, including prodrugs of naltrexone and/or prodrugs of naloxone, in a total amount of about of between about 0.1% and about 95% by weight of the composition. For example, one or more opioid agonists or agonist-antagonists may be present in the amount by weight of: about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 4%, about 5%, about 6%, about 7%, about 8%, about 9%, about 10%, about 11%, about 12%, about 13%, about 14%, about 15%, about 20%, about 25%, about 30%, about 35%, about 40%, about 45%, about 50%, about 55%, about 60%, about 65%, about 70%, about 75%, about 80%, about 85%, about 90% or about 95%.

In another embodiment, a single dosage unit comprises a therapeutically effective amount or a therapeutically and/or prophylactically effective amount of an opioid agonist or opioid agonist-antagonist or prodrugs thereof, such as buprenorphine or prodrugs of buprenorphine. The term "therapeutically effective amount" or "therapeutically and/or prophylactically effective amount" as used herein refers to an amount of compound or agent that is sufficient to elicit the required or desired therapeutic and/or prophylactic response, as the particular treatment context may require. Single dosage unit as used herein includes individual patches, sachets containing a single dose, metered pumps designed to dispense a predetermined quantity of material for application to the skin as well as other means for dispensing a single or multiple doses for application to the skin.

It will be understood that a therapeutically and/or prophylactically effective amount of a drug for a subject is dependent *inter alia* on the body weight of the subject as well as other factors known to a person of ordinary skill in the art. A "subject" herein to which a therapeutic agent or composition thereof can be administered includes mammals such as a human of either sex and of any age, and also includes any nonhuman animal, particularly a domestic, farm or companion animal; illustratively a cat, cow, pig, dog or a horse as well as laboratory animals such as guinea pigs and primates.

The terms "treat", "treated", "treating" and "treatment" are to be broadly understood as referring to any response to, or anticipation of, a medical condition in a mammal, particularly a human, and includes but is not limited to:
(i) inhibiting the medical condition, i.e., arresting, slowing or delaying the on-set, development or progression of the medical condition; or
(ii) relieving the medical condition, i.e., causing regression of the medical condition.

In one embodiment, a therapeutically effective amount of an opioid agonist or agonist-antagonist, such as buprenorphine, is administered transdermally in an abuse-resistant or abuse deterrent formulation to treat a medical condition selected from the group consisting of: opioid dependence, alcohol dependence, polydrug addiction, pain, cocaine addiction, eating disorders (e.g., binge eating) and treatment-resistant depression.

Pain can include nociceptive pain, such as somatic pain and visceral pain, and noncociceptive pain, such as neuropathic pain, sympathetic pain, psychogenic pain and idiopathic pain. Pain also include chronic and acute pain. Non-limiting examples of pain include fibromyalgia, chronic back pain (both deep and superficial somatic pain), chronic pancreatitis, chronic acute hepatitis, gallstone, appendicitis, post-herpetic neuralgia, trigeminal neuralgia, phantom limb pain, diabetic neuropathy, carpal tunnel syndrome, sciatica, pudendal neuralgia, central pain syndrome, spinal cord injury, post-surgical pain, cancer, degenerative disk disease, osteoporosis, peripheral neuropathy, herpes zoster (shingles), lupus, reflex sympathetic dystrophy, headaches (migraines, tension and cluster), temporomandibular disorders, such as temporomandibular joint syndrome, myofacial pain, internal derangement of the joint, and degenerative joint diseases, such as osteoarthritis and rheumatoid arthritis.

Eating disorders can include anorexia nervosa, bulimia nervosa, binge eating disorder (BED), compulsive overeating, purging disorder, rumination, diabulimia, food maintenance, eating disorders not otherwise specified (EDNOS), pica, night eating syndrome and orthorexia nervosa.

In one embodiment, the pharmaceutical composition is administered once daily to a subject in need thereof. In a further embodiment, the pharmaceutical composition comprising an opioid agonist or agonist-antagonist, such as buprenorphine, is administered twice daily to a subject in need thereof. In a further embodiment, the pharmaceutical composition is administered more than twice daily, such as three, four, five, six, seven or eight times daily.

In a further embodiment, the pharmaceutical composition is administered every second day, every third day, every fourth day, every fifth, every sixth day, or once weekly.

### Combination with Non-Opioid Agents

In one embodiment, the pharmaceutical composition containing the opioid or opioid prodrug can also be combined with an optional second non-opioid pharmacologically active agent for the treatment of pain and/or polydrug abuse, including, for example, a cannabinoid (agonist, antagonist, or inverse agonist), bupropion, hydroxybupropion, nicotine, nornicotine, varenicline, doxepin, acetaminophen, aspirin, or another non-steroidal anti-inflammatory drug. The cannabinoid could consist of one or more of the drugs or prodrugs as described in U.S. Pat. App. Nos. 11/157,034, filed June 30, 2005, published as U.S. 2005/0266061, 12/182,974, filed July 30, 2008, published as U.S. 2009/036523 and 12/326,036, filed December 1, 2008, published as U.S. 2009/0143462. The previous listing of suitable compounds for use as an optional second non-opioid pharmacologically active agent is not meant to be exhaustive, as a person of ordinary skill in the art would understand that other compounds (such as those found in the Merck Index, Thirteenth Edition and the Physicians Desk Reference, 58th ed.) would be suitable for use as the optional second non-opioid pharmacologically active agent in the invention disclosed herein.

### Pharmaceutical Excipients

The pharmaceutical compositions described herein can, if desired, include one or more pharmaceutically acceptable excipients. The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition. Excipients include, by way of illustration and not limitation, solvents, thickening agents, penetration enhancers, wetting agents, lubricants, emollients, substances added to mask or counteract a disagreeable odor or flavor, fragrances, and substances added to improve appearance or texture of the composition. Any such excipients can be used in any composition of the present disclosure. The foregoing list of excipients is not meant to be exhaustive but merely illustrative as a person of ordinary skill in the art would recognize that additional excipients could be utilized.

Compositions described herein containing excipients can be prepared by any technique known to a person of ordinary skill in the art of pharmacy, pharmaceutics, drug delivery, pharmacokinetics, medicine or other related discipline that comprises admixing one or more excipients with a therapeutic agent.

In one embodiment, the composition may comprise one or more penetration enhancing agent for transdermal drug delivery. Non-limiting examples of penetration enhancing agents include C8-C22 fatty acids such as isostearic acid, octanoic acid and oleic acid; C8-C22 fatty alcohols such as oleyl alcohol and lauryl alcohol; lower alkyl esters of C8-C22 fatty acids such as ethyl oleate, isopropyl myristate, butyl stearate, and methyl laurate; di(lower)alkyl esters of C6-C22 diacids such as diisopropyl adipate; monoglycerides of C8-C22 fatty acids such as glyceryl monolaurate; tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; N-alkylpyrrolidone and terpenes. Additional penetration enhancers suitable for use can also be found in United States Pat. App. No. 10/032,163, filed December 21, 2001, published as 2002/0111377 A1 and in Thong, et al., "Percutaneous Penetration Enhancers: An Overview," Skin Pharmacology and Physiology, 20:272-828 (2007).

The penetration enhancing agent is present in an amount sufficient to provide the desired physical properties and skin penetration profile for the composition. Illustratively, one or more pharmaceutically acceptable penetration enhancer can be present in a total amount by weight of the composition of about 0.1%, about 0.2%, about 0.3%, about 0.4%, about 0.5%, about 1.0%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10.0%, about 10.5%, about 11.0%, about 11.5%, about 12.0%, about 12.5%, about 13.0%, about 13.5%, about 14.0%, about 14.5% or 15.0%. As a further illustration, one or more pharmaceutically acceptable penetration enhancer is present in a total amount by weight between about 0.1% and about 15%; between about 0.1% and about 10%; between about 0.5% and about 10% or between about 3% and about 8%.

As a further illustration, one or more pharmaceutically acceptable penetration enhancer is present in a total amount by weight between about 1% and about 10%, between about 2% and about 10%, between about 3% and about 10%, between about 4% and about 10%, between about 5% and about 10%, between about 6% and about 10%, between about 7% and about 10%, between about 8% and about 10%, between about 9% and about 10%, between about 1% and about 9%, between about 2% and about 9%, between about 3% and about 9%, between about 4% and about 9%, between about 5% and about 9%, between about 6% and about 9%, between about 7% and about 9%, between about 8% and about 9%, between about 1% and about 8%, between about 2% and about 8%, between about 3% and about 8%, between about 4% and about 8%, between about 5% and about 8%, between about 6% and about 8%, between about 7% and about 8%, between about 1% and about 7%, between about 2% and about 7%, between about 3% and about 7%, between about 4% and about 7%, between about 5% and about 7%, between about 6% and about 7%, between about 1% and about 6%, between about 2% and about 6%, between about 3% and about 6%, between about 4% and about 6%, between about 5% and about 6%, between about 1% and about 5%, between about 2% and about 5%, between about 3% and about 5%, between about 4% and about 5%, between about 1% and about 4%, between about 2% and about 4%, between about 3% and about 4%, between about 1% and about 3%, between about 2% and about 3% and between about 1% and about 2%.

The thickening agents (aka gelling agents) used herein may include anionic polymers such as polyacrylic acid (CARBOPOL® by Noveon, Inc., Cleveland, Ohio), carboxypolymethylene, carboxymethylcellulose and the like, including derivatives of Carbopol® polymers, such as Carbopol® Ultrez 10, Carbopol® 940, Carbopol® 941, Carbopol® 954, Carbopol® 980, Carbopol® 981, Carbopol® ETD 2001, Carbopol® EZ-2 and Carbopol® EZ-3, and other polymers such as Pemulen® polymeric emulsifiers, and Noveon® polycarbophils. Additional thickening agents, enhancers and adjuvants may generally be found in Remington's The Science and Practice of Pharmacy as well as the Handbook of Pharmaceutical Excipients, Arthur H. Kibbe ed. 2000. Thickening agents or gelling agents are present in an amount sufficient to provide the desired rheological properties of the composition. Illustratively, one or more pharmaceutically acceptable thickening agent or gelling agent are present in a total amount by weight of about 0.1%, about 0.25%, about 0.5%, about 0.75%, about 1%, about 1.25%, about 1.5%, about 1.75%, about 2.0%, about 2.25%, about 2.5%, about 2.75%, about 3.0%, about 3.25%, about 3.5%, about 3.75%, about 4.0%, about 4.25%, about 4.5%, about 4.75%, about 5.0%, about 5.25%, about 5.5%, about 5.75%, about 6.0%, about 6.25%, about 6.5%, about 6.75%, about 7.0%, about 7.25%, about 7.5%, about 7.75%, about 8.0%, about 8.25%, about 8.5%, about 8.75%, about 9.0%, about 9.25%, about 9.5%, about 9.75%, about 10%, about 11%, about 11.5%, about 12%, about 12.5%, about 13%, about 13.5%, about 14%, about 14.5% or about 15%. As a further illustration, one or more pharmaceutically acceptable thickening or gelling agent are present in a total amount by weight between about 0.1% and about 15%; between about 0.5% and about 5% or between about 1% and about 3%.

In one embodiment a neutralizing agent is optionally present to assist in forming a gel. Suitable neutralizing agents include sodium hydroxide (e.g., as an aqueous mixture), potassium hydroxide (e.g., as an aqueous mixture), ammonium hydroxide (e.g., as an aqueous mixture), triethanolamine, tromethamine (2-amino 2-hydroxymethyl-1, 3 propanediol), aminomethyl propanol (AMP), tetrahydroxypropyl ethylene diamine, diisopropanolamine, Ethomeen C-25 (Armac Industrial Division), Di-2 (ethylhexyl) amine (BASF-Wyandotte Corp., Intermediate Chemicals Division), triamylamine, Jeffamine D-1000 (Jefferson Chemical Co.), b-Dimethylaminopropionitrite (American Cyanamid Co.), Armeen CD (Armac Industrial Division), Alamine 7D (Henkel Corporation), dodecylamine and morpholine. The neutralizing agent is present in an amount sufficient to increase viscosity and form a gel or gel-like composition which is suitable for contact with the skin of a mammal. Illustratively, one or more pharmaceutically acceptable neutralizing agent is present in a total amount by weight of about 0.001%, about 0.0015%, about 0.01%, about 0.015%, about 0.1%, about 0.2%, about 0.3%, about 0.4%, about 0.5%, about 0.6%, about 0.7%, about 0.8%, about 0.9%, about 1.0%, 1.1%, about 1.2%, about 1.3%, about 1.4%, about 1.5%, about 1.6%, about 1.7%, about 1.8%, about 1.9%, about 2.0%, 2.1%, about 2.2%, about 2.3%, about 2.4%, about 2.5%, about 2.6%, about 2.7%, about 2.8%, about 2.9%, about 3.0%, about 3.1%, about 3.2%, about 3.3%, about 3.4%, about 3.5%, about 3.6%, about 3.7%, about 3.8%, about 3.9%, about 4.0%, about 4.1%, about 4.2%, about 4.3%, about 4.4%, about 4.5%, about 4.6%, about 4.7%, about 4.8%, about 4.9%, about 5.0%, about 5.1%, about 5.2%, about 5.3%, about 5.4%, about 5.5%, about 5.6%, about 5.7%, about 5.8%, about 5.9%, about 6.0%, about 6.1%, about 6.2%, about 6.3%, about 6.4%, about 6.5%, about 6.6%, about 6.7%, about 6.8%, about 6.9% or about 7.0%. As a further illustration, one or more pharmaceutically acceptable neutralizing agent is present in a total amount by weight between about 0.1% and about 7% or between about 1% and about 5%. In one embodiment, a solution of sodium hydroxide is used, such as, *e.g.,* 0.01 N sodium hydroxide solution, 0.02 N sodium hydroxide solution, 0.025 N sodium hydroxide solution, 0.05 N sodium hydroxide solution, 0.075 N sodium hydroxide solution, 0.1 N sodium hydroxide solution, 0.2 N sodium hydroxide solution, 0.5 N sodium hydroxide solution, 1.0 N sodium hydroxide solution, 1.5 N sodium hydroxide solution, 2.0 N sodium hydroxide solution, 10.0 N sodium hydroxide solution or any other suitable solution for providing an amount sufficient of the aqueous sodium hydroxide to form the desired gel. In one embodiment, the composition results from combining a gelling agent with a neutralizing agent, such as about 1% to about 10% (wt/wt) 0.025 N sodium hydroxide, while in another embodiment about 0.1% to about 1% (wt/wt) 0.25 N sodium hydroxide is used. Of course, other suitable neutralizing agents can be used, as can other concentrations and amounts of aqueous sodium hydroxide, so long as there is a sufficient amount of OH⁻ ions to assist in the formation of a gel.

In a further embodiment, the formulation is a gel, an ointment, a cream or a patch and comprises a buprenorphine prodrug, optionally one or more penetration enhancing agent, thickening agent, lower alcohol, such as ethanol or isopropanol; or water. In another embodiment, the formulation is a gel, an ointment, a cream or a patch, further comprised of sodium hydroxide or triethanolamine or potassium hydroxide, or a combination thereof, in an amount sufficient, as is known in the art, to assist the gelling agent in forming a gel suitable for contact with the skin of a mammal.

Compositions described herein optionally comprise one or more pharmaceutically acceptable wetting agents as excipients. Non-limiting examples of surfactants that can be used as wetting agents in compositions of the disclosure include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10 and octoxynol 9, poloxamers (polyoxyethylene and polyoxypropylene block copolymers), polyoxyethylene fatty acid glycerides and oils, for example polyoxyethylene (8) caprylic/capric mono- and diglycerides (e.g., Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (e.g., Tween™ 80 of ICI), propylene glycol fatty acid esters, for example propylene glycol laurate (e.g., Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, about 0.4% to about 10% or about 0.5% to about 5% of the total weight of the composition. Illustratively, one or more pharmaceutically acceptable wetting agents are present in a total amount by weight of about 0.25%, about 0.5%, about 0.75%, about 1%, about 1.25%, about 1.5%, about 1.75%, about 2.0%, about 2.25%, about 2.5%, about 2.75%, about 3.0%, about 3.25%, about 3.5%, about 3.75%, about 4.0%, about 4.25%, about 4.5%, about 4.75%, about 5.0%, about 5.25%, about 5.5%, about 5.75%, about 6.0%, about 6.25%, about 6.5%, about 6.75%, about 7.0%, about 7.25%, about 7.5%, about 7.75%, about 8.0%, about 8.25%, about 8.5%, about 8.75%, about 9.0%, about 9.25%, about 9.5%, about 9.75% or about 10%.

Compositions described herein optionally comprise one or more pharmaceutically acceptable lubricants (including anti-adherents and/or glidants) as excipients. Suitable lubricants include, either individually or in combination, glyceryl behenate (e.g., Compritol™ 888); stearic acid and salts thereof, including magnesium (magnesium stearate), calcium and sodium stearates; hydrogenated vegetable oils (e.g., Sterotex™); colloidal silica; talc; waxes; boric acid; sodium benzoate; sodium acetate; sodium fumarate; sodium chloride; DL-leucine; PEG (*e.g.,* Carbowax™ 4000 and Carbowax™ 6000); sodium oleate; sodium lauryl sulfate; and magnesium lauryl sulfate. Such lubricants, if present, constitute in total about 0.1% to about 10%, about 0.2% to about 8% or about 0.25% to about 5% of the total weight of the composition. Illustratively, one or more pharmaceutically acceptable lubricants are present in a total amount by weight of about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0%, about 2.1%, about 2.2%, about 2.3%, about 2.4%, about 2.5%, about 2.6%, about 2.7%, about 2.8%, about 2.9%, about 3.0%, about 3.1%, about 3.2%, about 3.3%, about 3.4%, about 3.5%, about 3.6%, about 3.7%, about 3.8%, about 3.9%, about 4.0%, about 4.1%, about 4.2%, about 4.3%, about 4.4%, about 4.5%, about 4.6%, about 4.7%, about 4.8%, about 4.9%, about 5.0%, about 5.1%, about 5.2%, about 5.3%, about 5.4%, about 5.5%, about 5.6%, about 5.7%, about 5.8%, about 5.9%, about 6.0%, about 6.1%, about 6.2%, about 6.3%, about 6.4%, about 6.5%, about 6.6%, about 6.7%, about 6.8%, about 6.9%, about 7.0%, about 7.1%, about 7.2%, about 7.3%, about 7.4%, about 7.5%, about 7.6%, about 7.7%, about 7.8%, about 7.9%, about 8.0%, about 8.1%, about 8.2%, about 8.3%, about 8.4%, about 8.5%, about 8.6%, about 8.7%, about 8.8%, about 8.9%, about 9.0%, about 9.1%, about 9.2%, about 9.3%, about 9.4%, about 9.5%, about 9.6%, about 9.7%, about 9.8%, about 9.9% or about 10.0%.

In another embodiment, the compositions described herein optionally comprise an emollient. Illustrative emollients include mineral oil, mixtures of mineral oil and lanolin alcohols, cetyl alcohol, cetostearyl alcohol, petrolatum, petrolatum and lanolin alcohols, cetyl esters wax, cholesterol, glycerin, glyceryl monostearate, isopropyl myristate, isopropyl palmitate, lecithin, allyl caproate, althea officinalis extract, arachidyl alcohol, argobase EUC, Butylene glycol dicaprylate/dicaprate, acacia, allantoin, carrageenan, cetyl dimethicone, cyclomethicone, diethyl succinate, dihydroabietyl behenate, dioctyl adipate, ethyl laurate, ethyl palmitate, ethyl stearate, isoamyl laurate, octanoate, PEG-75 lanolin, sorbitan laurate, walnut oil, wheat germ oil super refined almond, super refined sesame, super refined soybean, octyl palmitate, caprylic/capric triglyceride and glyceryl cocoate.

An emollient, if present, is present in the compositions described herein in an amount of about 1% to about 30%, about 3% to about 25% or about 5% to about 15% by weight. Illustratively, one or more emollients are present in a total amount by weight of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29% or about 30%.

In one embodiment, the compositions described herein comprise an antioxidant. Illustrative antioxidants include citric acid, butylated hydroxytoluene (BHT), ascorbic acid, glutathione, retinol, α-tocopherol, β- carotene, α-carotene, ubiquinone, butylated hydroxyanisole, ethylenediaminetetraacetic acid, selenium, zinc, lignan, uric acid, lipoic acid and N-acetylcysteine. An antioxidant, if present, is present in the compositions described herein in the amount of less than about 1% by weight. Illustratively, one or more antioxidants are present in the total amount of about 0.025%, about 0.05%, about 0.075%, about 0.1%, 0.125%, about 0.15%, about 0.175%, about 0.2%, 0.225%, about 0.25%, about 0.275%, about 0.3%, 0.325%, about 0.35%, about 0.375%, about 0.4%, 0.425%, about 0.45%, about 0.475%, about 0.5%, 0.525%, about 0.55%, about 0.575%, about 0.6%, 0.625%, about 0.65%, about 0.675%, about 0.7%, 0.725%, about 0.75%, about 0.775%, about 0.8%, 0.825%, about 0.85%, about 0.875%, about 0.9%, 0.925%, about 0.95%, about 0.975% or about 1.0% by weight. As a further illustration, one or more antioxidants are present in the total amount by weight of between about 0.01% and about 1.0%; between about 0.05% and about 0.5% or between about 0.05% and about 0.2%.

In one embodiment, a composition comprises an antimicrobial preservative. Illustrative anti-microbial preservatives include acids, including but not limited to, benzoic acid, phenolic acid, sorbic acids, alcohols, benzethonium chloride, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, propylparaben, sodium propionate or thimerosal. The anti-microbial preservative, if present, is present in an amount of about 0.1% to about 5%, about 0.2% to about 3% or about 0.3% to about 2% by weight; for example about 0.2%, 0.4%, 0.6%, 0.8%, 1%, 1.2%, 1.4%, 1.6%, 1.8%, 2%, 2.4%, 2.6%. 2.8%, 3.0%, 3.2%, 3.4%, 3.6%, 3.8%, 4%, 4.2%, 4.4%, 4.6%, 4.8% or 5%.

Compositions described herein optionally compromise one or more emulsifying agents. The term "emulsifying agent" refers to an agent capable of lowering surface tension between a non-polar and polar phase and includes compounds defined as "self emulsifying" agents. Suitable emulsifying agents can come from any class of pharmaceutically acceptable emulsifying agents including carbohydrates, proteins, high molecular weight alcohols, wetting agents, waxes and finely divided solids. The emulsifying agent, if present, is present in a composition in a total amount of about 1% to about 15%, about 1% to about 12%, about 1% to about 10%, or about 1% to about 5% by weight of the composition. Illustratively, one or more emulsifying agents are present in a total amount by weight of about 1%, about 2%, about 3%, about 4%, about 5%, about 6%, about 7%, about 8%, about 9%, about 10%, about 11%, about 12%, about 13%, about 14% or about 15%.

In another embodiment, the water immiscible solvent comprises propylene glycol, and is present in a composition in an amount of about 1% to about 99% by weight of the composition; for example about 1%, about 2%, about 3%, about 4%, about 5%, about 6%, about 6%, about 7%, about 8%, about 9%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 99%.

Composition described herein may optionally comprise one or more alcohols. In a further embodiment, the alcohol is a lower alcohol. As used herein, the term "lower alcohol," alone or in combination, means a straight-chain or branched-chain alcohol moiety containing one to six carbon atoms. In one embodiment, the lower alcohol contains one to four carbon atoms, and in another embodiment the lower alcohol contains two or three carbon atoms. Examples of such alcohol moieties include ethanol, ethanol USP (i.e., 95% v/v), n-propanol, isopropanol, n-butanol, isobutanol, sec-butanol, and tert-butanol. As used herein, the term "ethanol" refers to C₂H₅OH. It may be used as dehydrated alcohol USP, alcohol USP or in any common form including in combination with various amounts of water. If present, the alcohol is present in an amount sufficient to form a composition which is suitable for contact with a mammal. Illustratively, one or more pharmaceutically acceptable alcohol is present in a total amount by weight of about 1%, about 2%, about 3%, about 4%, about 5%, about 6%, about 7%, about 8%, about 9%,about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97% or about 98%. As a further illustration, one or more pharmaceutically acceptable alcohol is present in a total amount by weight between about 1% and about 98%; between about 10% and about 95%; between about 25% and about 75%; between about 35% and about 70% or between about 40% and about 50%.

In one embodiment a pressure sensitive adhesive is optionally used to assist in affixing a patch containing an opioid to be transdermally delivered to the subject. In a further embodiment, the pressure sensitive adhesive is present in a total amount by weight between about 1% and about 99.9%; between about 50% and about 99.9% and between about 75% and about 99.9%. In a further embodiment the pressure sensitive adhesive layer is a mixture of two or more pressure sensitive adhesives. In another embodiment, the pressure sensitive adhesive is a mixture of Bio-PSA silicone adhesive 7-4201 and Duro-Tak 87-9301 (manufactured by Dow Corning Corporation, Medical Products, Midland, Mich and the National Starch and Chemical Company, respectively) which are mixed in a ratio of about 10:1 (7-4202:87-9301).

Non-limiting examples of pressure sensitive adhesives include polymer and copolymers of polyacrylates, polysiloxanes, polyisobutylene, polyisoprene, polybutadiene, ethylene-vinyl acetate and styrenic block polymers, such as styrene-isoprene-styrene block copolymer, styrene-butadiene-styrene copolymer, styrene-ethylenebutene-styrene copolymers, styrene-ethylene/propylene-styrene copolymers and di-block analogs thereof. Examples of polyacrylates include, but are not limited to, acrylic acids, alkyl acrylates and methacrylates; for example, acrylic acid, methacrylic acid, methoxyethyl acrylate, ethyl acrylate, butyl acrylate, butyl methacrylate, hexyl acrylate, hexyl methacrylate, 2-ethylbutyl acrylate, 2-ethylbutyl methacrylate, isooctyl acrylate, isooctyl methacrylate, 2-ethylhexyl acrylate, 2-ethylhexyl methacrylate, decyl acrylate, decyl methacrylate, dodecyl acrylate, dodecyl methacrylate, tridecyl acrylate, tridecyl methacrylate, hydroxyethyl acrylate, hydroxypropyl acrylate, acrylamide, dimethylacrylamide, acrylonitrile, dimethylaminoethyl acrylate, dimethylaminoethyl methacrylate, tert-butylaminoethyl acrylate, tert-butylaminoethyl methacrylate, methoxyethyl acrylate, methoxyethyl methacrylate, vinylacetate/ethylene acrylate and the like. Additional examples of appropriate acrylic adhesives suitable in the practice of the invention are described in Satas, "Acrylic Adhesives," Handbook of Pressure-Sensitive Adhesive Technology, 2nd ed., pp. 396-456 (D. Satas, ed.), Van Nostrand Reinhold, New York (1989).

Other useful pressure sensitive adhesives (PSA) can include mixtures of different polymers or mixtures of polymers such as synthetic rubber polyisobutylene (PIB), The PIB adhesives normally include a tackifier such as polybutene oil and resins such as the ESCOREZ® resins available from Exxon Chemical. Other useful rubber-based pressure-sensitive adhesives include hydrocarbon polymers such as natural and synthetic polyisoprene, polybutylene and polyisobutylene, styrene/butadiene polymers styrene-isoprene-styrene block copolymers, hydrocarbon polymers such as butyl rubber, halogen-containing polymers such as polyacrylic-nitrile, polytetrafluoroethylene, polyvinylchloride, polyvinylidene chloride, and polychlorodiene, and other copolymers thereof. Additional suitable pressure sensitive adhesives can be found in U.S. Pat. No. 7,867,986. Polyisobutylene polymers are available commercially under the trademark name VISTANEX® from Exxon Chemical.

Silicone-based pressure sensitive adhesives are also suitable for use in additional embodiments described herein. Suitable silicone-based pressure-sensitive adhesives can include those described in Sobieski, et al., "Silicone Pressure Sensitive Adhesives," Handbook of Pressure-Sensitive Adhesive Technology, 2nd ed., pp. 508-517 (D. Satas, ed.), Van Nostrand Reinhold, New York (1989). Other useful silicone-based pressure sensitive adhesives are described in the following U.S. Patents: U.S. Pat. Nos. 4,591,622; 4,584,355; 4,585,836; and 4,655,767. Suitable silicone-based pressure-sensitive adhesives are commercially available and include the silicone adhesives sold under the trademarks BIO-PSA 7-4503, BIO-PSA 7-4603, BIO-PSA 7-4301, 7-4202, 7-4102, 7-4106, and BIO-PSA 7-4303 by Dow Corning Corporation, Medical Products, Midland, Mich. The commercially available silicones are sold under the trademark of BIO-PSA such as Bio-PSA 7-4102, 7-4202, 7- 4302, 7-4101, 7-4201, 7-4301, 7-4303, 7-4503, 7-4603 by Dow Corning Cooperation. In one embodiment, amine-compatible Bio-PSA silicone adhesives are preferred. In a further embodiment, the preferred amine-compatible Bio-PSA silicone adhesive 7-4202 was employed in combination with acrylic adhesive such as Duro-tak 87-9301 manufactured by National Starch and Chemical Company.

In a further embodiment water is separately added to the composition. The amount of water separately added to a formulation is exclusive of the amount of water independently present in the composition from any other component (e.g., alcohol, neutralizing agent). Water is present in an amount sufficient to form a composition which is suitable for administration to a mammal. Illustratively, water can be separately added by weight in an amount of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% about 91%, about 92%, about 93%, about 94%, about 95%, about 96%, about 97% or about 98%. As a further illustration, water can be separately added by weight in an amount between about 1% and about 98%; between about 10% and about 70%; between about 10% and about 40%; between about 10% and about 30%; between about 20% and about 30% or between about 25% and about 30%.

In a further embodiment, the pharmaceutical composition is substantially free of water. In yet a further embodiment, the pharmaceutical composition is anhydrous.

### Pharmaceutical Dosage Forms

In one embodiment, compositions described herein are suitable for transdermal administration. In another embodiment, transdermally administrable compositions are adapted for administration in and/or around the abdomen, back, chest, legs, arms, scalp or other suitable skin surface and maybe formulated as patches, ointments, creams, suspensions, lotions, pastes, gels, sprays, foams, oils or other form suitable for transdermal administration.

In another embodiment, compositions described herein which are transdermally administrable include opioid prodrugs, including prodrugs of buprenorphine, placed in a propylene glycol or gel formulation for administration as patches, ointments, creams, suspensions, lotions, pastes, gels, sprays, foams, oils or other form suitable for transdermal administration.

### Gel Formulations

Alcoholic gels and emulsions have become more popular for systemic delivery of pharmacologically active agents. Testosterone and estradiol products are examples of products on the market now which are gaining market share relative to competitive patch products. Typically patches have been the mainstay for systemic transdermal drug delivery. Ironically, the original transdermal dosage form was a nitroglycerin ointment that was measured out to provide the correct dose. For modern transdermal systemic delivery, many gels and creams have unit dose packaging and calibrated pump dispensers designed to provide the correct dose for application to the skin of the subject. Systemic gel treatments take advantage of the fact that much larger skin surface areas can be covered with the drug, which will improve the chances of therapeutic blood level success. Patches can usually only be made at a maximum area of 50 cm²; however, this is not a desirable size. Alcoholic gels can be made and can optionally include a gelling agent such as ethyl cellulose or a Carbopol. Optionally, appropriate levels of penetration enhancers can be incorporated into the gel.

Additional embodiments which can be prepared include the following compositions:

| Gel formulation used for rubbing into skin | |
|---|---|
| 92% | absolute ethanol, USP/NF |
| 5% | propylene glycol |
| 2% | Klucel® hydroxypropylcellulose |
| 1% | buprenorphine or prodrug of buprenorphine |

| Gel formulation | |
|---|---|
| 92% | absolute ethanol, USP/NF |
| 5% | ethylene glycol, USP |
| 2% | Klucel® hydroxypropylcellulose |
| 1% | buprenorphine or prodrug of buprenorphine |

| Gel formulation | |
|---|---|
| 91.75% | absolute ethanol, USP/NF |
| 5.0% | ethylene glycol, USP |
| 1% | buprenorphine or prodrug of buprenorphine |
| 1.25% | Di-2 (ethylhexyl) amine |
| 0.5% | Carbopol 980®, NF |
| 0.5% | isopropyl myristate, USP/NF |

### Patch Formulation

The compounds and pharmaceutical compositions described herein are suitable for use in transdermal delivery devices such as patches and the like. For example, the compounds and compositions described herein are suitable for use in a membrane-modulated transdermal delivery system. In this system, the reservoir containing the compound to be transdermally administered to the patient is encapsulated in a shallow compartment molded from a drug impermeable backing and a rate controlling polymeric membrane through which the compound to be delivered passes in a controlled manner. In one embodiment, the external surface of the membrane has a thin layer of a drug-compatible, hypoallergenic adhesive polymer (e.g., silicone or polyacrylate adhesive) which is applied to achieve intimate contact of the transdermal system with the skin.

The compounds and pharmaceutical compositions described herein are also suitable for use in adhesive-diffusion controlled transdermal systems. In these embodiments, the drug reservoir is formulated by directly dispersing the drug (or drugs) to be delivered in an adhesive polymer and then spreading the medicated adhesive onto a flat sheet of drug-impermeable backing membrane to form a thin drug reservoir layer. Optionally, on top of the drug reservoir layer, additional layers of non-medicated rate controlling adhesive polymer of constant thickness are placed to produce an adhesive diffusion-controlled drug-delivery system. Also, optionally a second adhesive layer can be added which can contain a drug substance whether or not it is to be transdermally delivered to the subject.

The compounds and pharmaceutical compositions described herein are also suitable for use in matrix dispersion-type systems. In these systems, the drug reservoir is formed by homogeneously dispersing the drugs in a hydrophilic or lipophilic polymer matrix, and the medicated polymer then is molded into a medicated disc with a defined surface area and controlled thickness. The disc then is glued onto an occlusive baseplate in a compartment fabricated from a drug-impermeable backing. The adhesive polymer is spread along the circumference to form a strip of adhesive rim around the medicated disc.

The compounds and pharmaceutical compositions described herein are also suitable for use in microreservoir systems. In these systems, the drug reservoir is formed by first suspending the drug particles in an aqueous solution of water-soluble polymer and then dispersing it homogeneously in a lipophilic polymer by high-shear mechanical force to form a large number of unleachable, microscopic spheres of drug reservoirs. This unstable dispersion is quickly stabilized by immediately cross-linking the which produces a medicated polymer disc with a constant surface area and fixed thickness. A transdermal therapeutic system is produced in which the medicated disc is positioned at the center and surrounded by an adhesive rim.

Patch formulations can be optimized using *in vitro* human skin diffusion testing prior to the selection of two or three patches for stability testing. In one embodiment, the drug and adhesive are formulated into one monolithic layer. The drug can be mixed with an adhesive (e.g. silicone type, available from Dow Corning and other manufacturers) in a solvent (e.g. methylene chloride or ethyl acetate). This drug mixture would then be extruded onto a polyester backing film to a uniform thickness of about 100 microns or greater with a precision wet film applicator. The solvent is allowed to evaporate in a drying oven and the resulting "patch" is trimmed to fit the diffusion cell donor chamber. Various patch formulations will be made until the desired steady-state flux rate and adhesive properties are obtained. Different adhesives can be tried, as well as varying the amount of adhesive in the formulation (Nalluri, Milligan et al. 2005). Suitable results have been obtained by making monolithic patches with DURO-TAK 387-2051, which is an acrylate-vinyl acetate non-curing pressure sensitive adhesive from the National Starch Chemical Company. Different solvents (e.g. isopropyl myristate, propylene glycol) can optionally be incorporated into the formulation in an attempt to optimize the delivery rate. In a further embodiment, reservoir patches can be made if it appears, for example, that the drugs are not compatible with a monolithic matrix patch formulation. In the reservoir system, the active ingredient(s) and any excipient(s) could be formulated into a gel and sealed between a release layer and an impermeable backing material such as polyester or other suitable material known to a person of skill in the art. Ethyl vinyl acetate membranes with acrylic adhesives have been found to be suitable.

Adhesive patch formulations can be prepared containing different loadings of a buprenorphine prodrug and optionally an opioid antagonist by using DURO-TAK adhesives (National Starch and Chemical Company, USA). Appropriate amounts of adhesive and drug can be sonicated for ten minutes, cast onto the release liner (9742 Scotchpak, 3M, St. Paul, MN) with a wet film applicator (Paul N. Gardner Company, Inc., Pompano Beach, FL) set at a 40 mil thickness, and kept at room temperature for one hour and then at 70°C in an oven for ten minutes (to remove any residual solvent). The patches would then be covered with backing membrane (CoTran 9722, 3M, St. Paul, MN), will be cut into appropriate sizes, and then can be stored in a desiccator for further study.

In further embodiments, additional adhesives which are suitable for preparing patch formulations and transdermal delivery devices such as patches include polyisobutylenes, acrylates, silicone and combinations of the foregoing. Additional adhesives can be found in United States Pat. App. Ser. No. 11/907,954, filed October 18, 2007, published as U.S. 2009/017102 A1.

In a further embodiment, the transdermal patch may optionally comprise more than one layer of opioid agonist, opioid agonist-antagonist or opioid antagonist or a prodrug of any of the foregoing. In a further embodiment, a respective layer may comprise an opioid agonist or an opioid agonist-antagonist alone or, optionally, in combination with an opioid antagonist. In yet a further embodiment, a respective layer may comprise an opioid antagonist separate from a layer comprising an opioid agonist or an opioid agonist-antagonist.

In another illustrative embodiment, the transdermal patch can be one which is capable of controlling the release of the buprenorphine or buprenorphine prodrug such that transdermal delivery of the buprenorphine or buprenorphine prodrug to the subject is substantially uniform and sustained over a period of about 6 hours, about 12 hours, about 24 hours, about 48 hours or about 7 days. Such transdermal patch which can be used in the practice of the methods described herein can take the form of an occlusive body. In practice, the occlusive body which includes the buprenorphine or buprenorphine prodrug is positioned on the subject's skin under conditions effective to transdermally deliver the buprenorphine or buprenorphine prodrug to the subject.

Other suitable patch formulations can be found in U.S. Pat. Appl. No. _filed on April 4, 2011 which claims priority to U.S. Provisional Appl. No. 61/320,526 filed April 2, 2010.

In addition to using the compounds and pharmaceutical compositions described herein in the transdermal delivery systems previously described, they are also suitable for use in conjunction with microneedles for transdermal drug delivery which create micrometer-scale transport pathways. Microneedles provide a minimally invasive means to transport molecules into the skin as the channels they create are extremely small on a clinical level. However, because the channels are much larger than even macromolecules, such channels should dramatically increase skin permeability.

Microneedles can be made from materials such as silicon, biodegradable polymers and stainless steel, as well as, other bio-compatible materials, and can be solid or hollow. Solid microneedles can be used to create holes in the skin, followed by application of a transdermal patch to the skin surface. Alternatively, solid microneedles can be first coated with a drug and then inserted into the skin. Hollow microneedles can also be used, to facilitate active fluid flow through the needle bore and into the skin. *See, e.g.,* Prausnitz, Adv. Drug. Deliv. Rev. 56 (2004) 581-587, for a review.

Numerous studies have demonstrated that solid microneedles can increase skin permeability by up to four orders of magnitude for compounds ranging in size from small molecules to proteins to nanoparticles (Henry et al., J. Pharm. Sci. 87 (1988) 922-925; McAllister et al., PNAS 100 (2003) 13755-13760; Lin et al., Pharm. Res. 18 (2001) 1787-1793 and Cormier et al., J. Control. Release. 97 (2004) 503-511). Hollow microneedles have also been shown to deliver insulin and reduce blood glucose levels (McAllister et al., PNAS 100 (2003) 13755-13760 and Martanto et al., Pharm. Res. 21 (2004) 947-952). Kaushik et al. studied the effects of pain associated with microneedle insertion in human volunteers and showed that the sensation was no more than that of a smooth surface applied to the skin or the "sensation of a piece of tape" applied to the skin (Kaushik et al., Anesth. Analg. 92 (2004) 502-504).

Suitable microneedle arrangements for use with the compounds and compositions described herein can be found in the foregoing references as well as in United States Patent Application No. 11/812,249, filed June 15, 2007, published as U.S. 2008/0008745 A1.

### Spray Formulation

In another embodiment, the opioid agonist (or prodrug thereof) or opioid agonist-antagonist (or prodrug thereof) can be delivered transdermally with a spray system. The metered dose spray would dispense a therapeutically effective dose to the skin. The dosing level could be achieved by creating a spray system that covers a desired skin area. The opioid agonist (or prodrug thereof) or opioid agonist-antagonist (or prodrug thereof) would absorb into the skin, forming a depot within the horny layer of this skin, and provide a sustained delivery of the opioid agonist (or prodrug thereof) or opioid agonist-antagonist (or prodrug thereof). In further embodiments, the metered dose spray may contain alcohols, fragrance, chemical enhancers (penetration enhancers), plasticizers, emollients, water, thickening agents, pH modifiers, fillers and preservatives as well as other suitable excipients.

### EXAMPLES

Examples not pertaining to the claimed invention are for illustrative purposes only.

### Example 1

Buprenorphine and the various buprenorphine prodrugs identified below were synthesized and assessed for permeation through human skin *in vitro :* 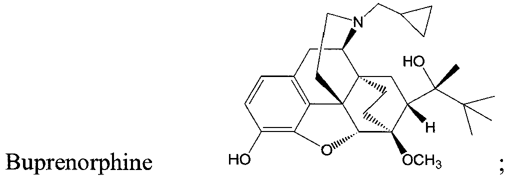 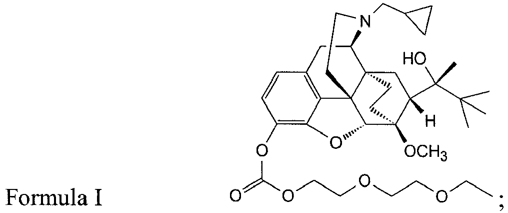 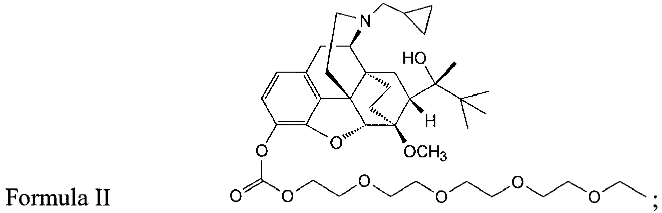 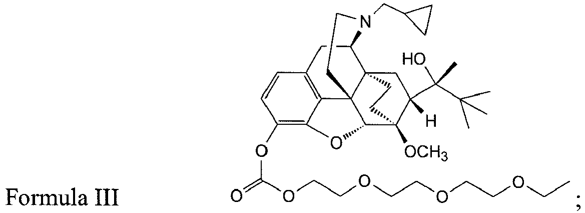 and

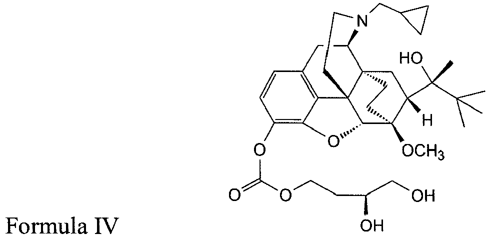

### Synthesis of buprenorphine base and buprenorphine prodrugs

### Synthesis of buprenorphine base

Buprenorphine hydrochloride (200 mg, 0.0004 mol) was suspended in about 10 mL of dichloromethane. Triethylamine (80 mg, 0.0008 mol) was added drop-by-drop. The solution was stirred until all material had dissolved. The solution was transferred to a 60-mL separatory funnel with rinses of dichloromethane. About 10 mL of water was added to the funnel and the contents agitated well by hand. The two phases were allowed to separate. The methylene chloride layer was removed and dried over anhydrous sodium sulfate for several hours. Methylene chloride was removed and combined with dichloromethane rinses of the sodium sulfate. The solvent was removed by a stream and nitrogen and the final product dried under vacuum.

### Synthesis of Formula I (Buprenorphine 3,6-dioxaoctyl carbonate)

Chloroformate solution was made by adding triphosgene (98.9 mg, 0.333 mmol) to a stirred solution of di(ethylene glycol) monoethyl ether (134.2 mg, 1 mmol) and dry triethylamine (101.2 mg, 0.139 mL, 1 mmol) in dry dichloromethane (2 mL) at 0°C under an argon atmosphere, and stirred for 1 h at 0°C.

Buprenorphine (125.1 mg, 0. 2675 mol) was dissolved in dry dichloromethane (20 mL) at 0°C under an argon atmosphere. Triethylamine (35.4 mg, 0.0488 mL, 0.35 mmol) was added, and after 5 min of stirring, chloroformate solution (0.8 mL) was added over a period of 30 seconds. After 5 min of stirring at 0°C, the solution was allowed to warm to ambient temperature. After 1 h, the reaction was quenched by adding saturated aqueous sodium bicarbonate solution at 0°C, and the aqueous layer was extracted with dichloromethane. The extract were dried over anhydrous sodium sulfate and concentrated under a reduced pressure. The residue was chromatographed on silica gel with DCM-MeOH (gradient 100:0,100:1,50:1,40:1,30:1) to afford 149.4 mg (89.0%) of Formula I (buprenorphine 3,6-dioxaoctyl carbonate) as an oil.

¹H NMR (400 MHz, CDCl₃): δ = 6.87(1H, d, J=8.2); 6.59(1H, d, J=8.2); 5.91(1H, s, H-5); 4.45(1H, d, J=1.6); 4.30-4.40 (2H, m); 3.77(2H, dd, J₁=5.1, J₂=4.5); 3.65-3.69(2H, m); 3.58-3.62(2H, m); 3.54(2H, q, J=7.0, CF₂CH₃); 3.48(s, 3H); 2.97-3.06(2H, m); 2.84-2.93(1H, m); 2.62(1H, dd, J₁=11.9, J₂=4.9); 2.22-3.38(4H, m); 2.12(1H, t, J=10.0); 1.98(1H, dt, J₁=12.7, J₂=5.6); 1.76-1.93(1H, m); 1.71(1H, dd, J₁=12.9, J₂=2.3); 1.35(3H, s, CH₃C), 1.31(1H, dd, J₁=13.1, J₂=9.4); 1.22(3H, t, J=7.0, CH₂CH₃); 1.03-1.12(1H, m); 1.03(9H, s, C(CH₃)₃); 0.74-0.85(1H, m); 0.62-0.72(1H, m); 0.43-0.55(2H, m, c-Pr); 0.07-0.16(2H, m, c-Pr).

### Synthesis of Formula II (Buprenorphine 3,6,9,12-tetraoxatetradecanyl carbonate)

Similar procedure to Formula I starting from tetra(ethylene glycol) monoethyl ether afforded Formula II (buprenorphine 3,6,9,12-tetraoxatetradecanyl carbonate) as an oil.

¹H NMR (400 MHz, CDCl₃): δ = 6.87(1H, d, J=8.0); 6.59(1H, d, J=8.0); 5.91(1H, s, H-5); 4.45(1H, d, J=1.8); 4.29-4.39 (2H, m); 3.76(2H, t, J=4.8); 3.63-3.69(10H, m); 3.57-3.62(2H, m); 3.53(2H, q, J=7.0, CH₂CH₃); 3.48(s, 3H); 2.97-3.07(2H, m); 2.83-2.94(1H, m); 2.62(1H, dd, J₁=11.9, J₂=4.9); 2.21-3.39(4H, m); 2.12(1H, t, J=9.9); 1.98(1H, dt, J₁=12.7, J₂=5.6); 1.76-1.93(1H, m); 1.71(1H, dd, J₁=12.9, J₂=2.3); 1.35(3H, s, CH₃C), 1.31(1H, dd, J₁=13.0, J₂=9.3); 1.21(3H, t, J=7.0, CH₂CH₃); 1.03-1.12(1H, m); 1.03(9H, s, C(CH₃)₃); 0.74-0.85(1H, m); 0.62-0.72(1H, m); 0.43-0.55(2H, m, c-Pr); 0.07-0.16(2H, m, c-Pr).

### Synthesis of Formula III (Buprenorphine 3,6,9-trioxaundecyl carbonate)

Similar procedure to Formula I starting from tri(ethylene glycol) monoethyl ether afforded Formula III (buprenorphine 3,6,9-trioxaundecyl carbonate) as an oil.

¹H NMR (400 MHz, CDCl₃): δ = 6.87(1H, d, J=8.1); 6.59(1H, d, J=8.1); 5.91(1H, s, H-5); 4.45(1H, d, J=1.8); 4.29-4.40 (2H, m); 3.76(2H, t, J=4.8); 3.64-3.69(6H, m); 3.58-3.63(2H, m); 3.53(2H, q, J=7.0, CH₂CH₃); 3.48(s, 3H); 2.97-3.06(2H, m); 2.83-2.93(1H, m); 2.62(1H, dd, J₁=11.9, J₂=4.9); 2.21-3.38(4H, m); 2.12(1H, t, J=10.0); 1.98(1H, dt, J₁=12.7, J₂=5.6); 1.76-1.93(1H, m); 1.71(1H, dd, J₁=13.0, J₂=2.4); 1.35(3H, s, CH₃C), 1.31(1H, dd, J₁=12.8, J₂=9.3); 1.21(3H, t, J=7.0, CH₂CH₃); 1.03-1.12(1H, m); 1.03(9H, s, C(CH₃)₃); 0.74-0.85(1H, m); 0.62-0.72(1H, m); 0.43-0.55(2H, m, c-Pr); 0.07-0.16(2H, m, c-Pr).

### Synthesis of Formula IV (Buprenorphine (3S)-3,4-dihydroxybutyl carbonate)

Chloroformate solution was made as follows: To a stirred solution of (4S)-(+)-4-(2-hydroxyethyl)-2,2-dimethyl-1,3-dioxolane (730.9 mg, 5.0 mmol) and triethylamine (521.1 mg, 0.718 mL, 5.15 mmol) in dry dichloromethane (10 mL) at 0°C under an argon atmosphere triphosgene (494.6 mg, 1.667 mmol) was added and stirring was continued for 1 h at 0°C. The solution of chloroformate was used directly in the next step.

Buprenorphine hydrochloride (1.313 g, 2.605 mol) was suspended in dry dichloromethane (20 mL) at 0°C under an argon atmosphere. Triethylamine (685 mg, 0.944 mL, 6.773 mmol) was added, and after 5 min of stirring, chloroformate solution (7.5 mL) was added over a period of 2 min. After 5 min of stirring at 0°C, the solution was allowed to warm to ambient temperature. After 1 h, the reaction was quenched by the addition of saturated aqueous sodium bicarbonate solution at 0°C, and the aqueous layer was extracted with dichloromethane. The extract was dried over anhydrous sodium sulfate and concentrated under a reduced pressure. The residue was chromatographed on silica gel with hexane - ethyl acetate (gradient 20:1,10:1,7:1,4:1,3:1) to afford 1.568 g (94.1%) of acetonide of buprenorphine (3S)-3,4-dihydroxybutyl carbonate as an oil.

Acetonide of buprenorphine (3S)-3,4-dihydroxybutyl carbonate (1.568 g) was dissolved in THF (55 mL) at 0°C and 1 N HCl (13.75 mL) was added with stirring. After 40 h at ambient temperature, the reaction mixture was diluted with water (100 mL), basified with triethylamine (1.736 g, 2.39 mL, 17.15 mmol), and extracted with dichloromethane (100 mL). The aqueous layer was extracted with dichloromethane (50 mL) with 0.2 mL of triethylamine. The extract was dried over anhydrous sodium sulfate and concentrated under a reduced pressure. The residue was chromatographed on silica gel with hexane - ethyl acetate (gradient 10:1,4:1,3:1,2:1,1:1,2:3, 1:2) to afford 1.273 g (86.6%) of Formula IV (buprenorphine (3S)-3,4-dihydroxybutyl carbonate) as a foam.

¹H NMR (400 MHz, CDCl₃): δ = 6.87(1H, d, J=8.1); 6.60(1H, d, J=8.1); 5.90(1H, s, H-5); 4.40-4.49 (2H, m); 4.32-4.40(1H, m); 3.84-3.93(1H, m); 3.69(1H, dd, J₁=11.1, J₂=2.9); 3.45-3.53(1H, m); 3.49(s, 3H); 2.98-3.06(2H, m); 2.84-2.93(1H, m); 2.63(1H, dd, J₁=11.9, J₂=4.9); 2.53(s br, 1H); 2.22-3.39(4H, m); 2.13(1H, t, J=9.9); 1.98(1H, dt, J₁=12.7, J₂=5.6); 1.75-1.94(4H, m); 1.72(1H, dd, J₁=13.0, J₂=2.4); 1.35(3H, s, CH₃C), 1.31(1H, dd, J₁=13.2, J₂=9.5); 1.03-1.12(1H, m); 1.03(9H, s, C(CH₃)₃); 0.74-0.85(1H, m); 0.62-0.73(1H, m); 0.43-0.55(2H, m, c-Pr); 0.07-0.16(2H, m, c-Pr)

### Proposed Synthesis of Formula V (Buprenorphine (3R)-3,4-dihydroxybutyl carbonate)

One possible route that Formula V could be synthesized is shown below.

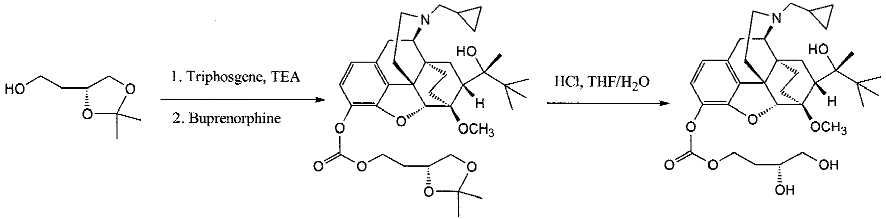

The starting material for the preparation of the R configuration of ALL00160a is available from Sigma-Aldrich as Catalog. No. 331074 (4R)-4-(2-Hydroxyethyl)-2,2-dimethyl-1,3-dioxolane.

### Plasma Stability Studies

An approximated 1 mg/mL stock solution of each prodrug was prepared in 100 µL of ethanol and 900 µL of acetonitrile. 10 µL of stock was spiked into 1 mL of plasma and vortexed. The samples were kept in a microcentrifuge tube and maintained at 37°C with a block heater and samples were obtained to analyze for bioconversion to parent drug.

### In vitro skin permeation studies

Equimolar concentrations of buprenorphine prodrugs were screened for enhanced permeation through human skin against buprenorphine base (BUP). Studies were run for 24 hours with sampling in 3 hour intervals. The cumulative quantity of drug collected in the receiver compartment was plotted as a function of time. The flux value for a given experiment was obtained from the slope of a steady state portion of the cumulative amount of drug permeated vs. time plot. Lag time was obtained from the x-intercept of the steady state portion of the cumulative amount of drug permeated vs. time plot.

As shown in the Table 1, Formulas I, III and IV all had enhanced permeation as compared to BUP. Likewise, there was higher skin disposition, similar lag times and enhanced permeation (at least a 2.75 fold increase in cumulative permeation) as compared to BUP. Formula IV had the highest plasma hydrolysis rate constant as compared to Formula I and II, suggesting that once the prodrug is in blood stream, it would be rapidly converted to the BUP. After completion of the permeation study, Formulas I, II and III were still 100% prodrug in the donor solution (24 h), while Formula IV was 9% buprenorphine in the donor solution.

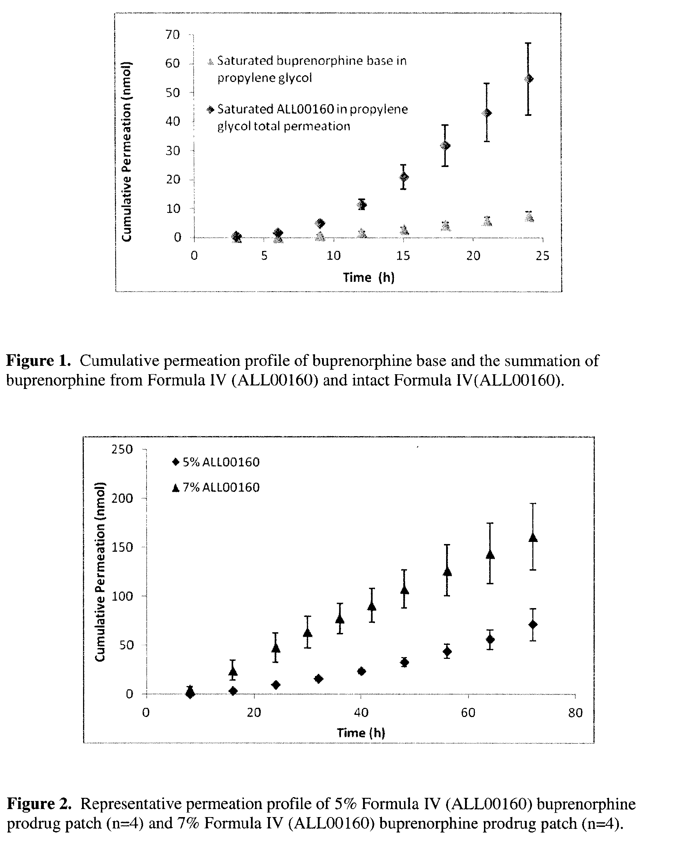

Figure 1 depicts the cumulative permeation profile of buprenorphine base and the summation of buprenorphine from Formula IV and intact Formula IV. The representative profile for Formula IV was almost completely free of buprenorphine base and only 5.7% of drug observed in the receiver solution after passing through the skin was Formula IV. [**TO DISCUSS]

**Table 1: Buprenorphine and buprenorphine prodrugs skin permeation data analysis and plasma hydrolysis.**

| **Compound (n)** | **Flux (nmol/cm²/h)** | **Lag time (h)** | **Cumulative permeation (nmol)** | **Skin Disposition (µmol/g)** | **Plasma hydrolysis (k) (h⁻¹)** |
|---|---|---|---|---|---|
| **BUP (12)** | 0.44 ± 0.14 | 9.2 ± 2.0 | 6.32 ± 2.16 | 0.62 ± 0.34 | --- |
| ***Formula I(3)** | 1.24 ± 0.23 | 7.7 ± 1.7 | 17.5 ± 4.0 | 1.0 ± 0.4 | -0.113 |
| **Formula II (4)** | 0.31 ± 0.26 | 7.4 ± 4.7 | 4.5 ± 1.9 | 4.5 ± 1.9 | -1.12 |
| ***Formula III (4)** | 1.74 ± 0.45 | 8.7 ± 0.6 | 23.9 ± 6.7 | 13.89 ± 12.21 | -0.37 |
| ***Formula IV (4)** | 3.85 ± 0.9 | 10.1 ± 0.7 | 54.8 ± 12.3 | 7.47 ± 4.95 | -0.79 |

| | | | | | |
|---|---|---|---|---|---|
| * Indicates a flux enhancement greater than 2. | | | | | |

### Example 2

**1.0 Purpose:** To develop a buprenorphine abuse deterrent patch to deliver therapeutic levels of buprenorphine transdermally using buprenorphine prodrug through human *skin in vitro.*

### METHODOLOGY

### 2.0 Skin details

Supplier: Cooperative Human Tissue Network
Storage: -20°C

### 3.0 Formulation of buprenorphine prodrug patches:

| **Patch 1 Formulation** | | **Patch 2 Formulation** | |
|---|---|---|---|
| **% w/w** | **Excipient** | **% w/w** | **Excipient** |
| 5 | Formula IV | 7 | Formula IV |
| 10 | Polyvinyl pyrollidone K29-32 | 10 | Polyvinyl pyrollidone K29-32 |
| 5 | Dipropylene glycol | 5 | Dipropylene glycol |
| 5 | Oleyl oleate | 5 | Oleyl oleate |
| 75 | 10:1 Dow Corning^{®} 4202:Durotak^{®} 9301 | 73 | 10:1 Dow Corning^{®} 4202:Durotak^{®} 9301 |
| 750 µL/g | Ethyl acetate | 450 µL/g | Ethyl acetate |
| 350 µL/g | Isopropyl alcohol | 200 µL/g | Isopropyl alcohol |

The patch was prepared by first dissolving Formula IV and polyvinyl pyrollidone K29-32 in ethyl acetate and isopropyl alcohol. Oleyl oleate and dipropylene glycol were added and mixed by vortexing. Both Durotak^{®} 900 A and Dow Corning^{®} 4302 polymers were weighed directly into the mixing vessel. The solution was again vortexed and sonicated to ensure content uniformity and to remove air from the matrix. Finally, the formulation was cast onto 3M™ Scotchpak™ 9744 release liner and cured at room temperature for 15 minutes and at 70°C for 30 minutes. 3M™ Scotchpak™ 1109 was used as the backing membrane.

### 4.0 In vitro skin permeation studies

Dermatomed human skin harvested from abdominoplasty and stored at -20° was used for the experiments. A PermeGear flow-through (In-Line, Hellertown, PA) diffusion cell system was used for the skin permeation studies.

Diffusion cells were kept at 32°C with a circulating water bath. Human epidermal skin was arranged in the diffusion cell with stratum corneum (upper layer of skin) facing the donor compartment. Permeation area of the skin was 0.95 cm². Data was collected from a human skin donor with four diffusion cells per treatment.

Receiver solution was 20% aqueous ethanol and flow rate was adjusted to 0.8 mL/h. Each cell had the respective 0.95 cm² patch applied to the skin.

Samples were collected into scintillation vials in 8 h increments for 72 h for the 5% patches, and 8 h increments for the first 24 h, then 6 h increments for the next 24-48 h, and 8 h increments for the remaining 48-72 h for the 7% patches. All the samples were stored at 4°C until analyzed. A 1 mL aliquot of the 20% aqueous ethanol diffusion samples was placed into HPLC vials and analyzed.

At the end of the experiment, the skin tissue was removed from the diffusion cell, rinsed with nanopure water, and blotted dry with a paper towel. The skin was tape stripped twice using book tape (Scotch™, 3M, St. Paul, MN) to remove drug formulation adhering to the tissue surface. The area of skin in contact with the drug was excised, chopped up and placed in a preweighed scintillation vial. Ten mL of acetonitrile was added to the vial and drug was extracted from the skin by shaking at room temperature overnight. The samples were analyzed by HPLC.

### 5.0 Analytical Method

| | |
|---|---|
| **Column** | Brownlee^{®} C₁₈ reversed phase Spheri 5 gm, (4.6 x 220 mm) column with a Brownlee^{®} C₁₈ reversed phase 7 µm (3.2 x 150 mm) guard column |
| **Mobile phase** | 90:10 acetonitrile:0.1% trifluoroacetic acid with 5% acetonitrile, pH=3 with triethylamine |
| **Flow rate** | 1.5 mL/min |
| **Wavelength** | 210 nm |
| **Injection volume** | 100 µL (diffusion samples and respective standards) 20 µL (skin samples, patch samples, and respective standards) |
| **Run time** | 5 min |
| **Retention times** | naltrexone = 3 min |
| | naloxone = 3 min |
| | Formula IV = 3.6 min |
| | buprenorphine = 4.2 min |
| **Naltrexone (skin)** | LOD in acetonitrile standards is 0.173 µg/mL or 0.507 nMoles/mL |
| | For a 20 mg skin sample (typical) and with extraction volume equal to 2 mL, then: 2 mL x 0.507 nM/mL = 1.14 nM or 1.14 nM/20mg skin = 0.057 nM/mG skin as LOD |
| **Naloxone (skin)** | LOD in ACN standards is 0.107 µg/mL or 0.327 nMoles/mL |
| | For a 20 mg skin sample (typical) and with extraction volume equal to 2 mL, then: 2 mL x 0.327 nM/mL = 0.654 nM, or 0.654 nM/20mg skin = 0.033 nM/mG skin as LOD |
| **Naltrexone (receiver fluid)** | 0.0346 µg/mL |
| **Naloxone (receiver fluid)** | 0.0215 µg/mL |

### 6.0 Data analysis

Cumulative quantity of drug collected in the receiver compartment was plotted as a function of time. The flux value for a given experiment was obtained from the slope of a steady state portion of the cumulative amount of drug permeated vs. time plot. Lag time was obtained from the x-intercept of the steady state portion of the cumulative amount of drug permeated vs. time plot. These values represent the data as total buprenorphine equivalents delivered in the form of buprenorphine and/or prodrug.

### RESULTS

Transtec^{®} and BuTrans^{®} deliver buprenorphine drug through the skin at a rate of 1.4 and 0.8 µg/cm²/h, respectively. As shown in Table 2, the formula containing 7% Formula IV delivered at a rate of 1.5 µg/cm²/h with a very short lag time, as compared to 1.4 µg/cm²/h that is delivered by the Transtec^{®} patch. Figure 2 depicts the total permeation of buprenorphine base from Formula IV through human skin from both 5% and 7% Formula IV. The study was performed over a 3 day period with steady state levels reached within the first day. Rapid conversion from Formula IV to parent buprenorphine was observed in human plasma (k = -0.79 h⁻¹); therefore, steady state levels of buprenorphine would be expected in human subjects with the first 24 h. The rate of delivery for 5% Formula IV formula was similar to the marketed BuTrans^{®} patch and the 7% Formula IV formula is equivalent to that of Transtec^{®}. The buprenorphine base equivalent formulas of buprenorphine prodrug (Formula IV) met or exceeded the currently available products with less drug loading concentration in the transdermal system.

**Table 2. Permeation data of 5% Formula IV buprenorphine prodrug patch (n=4) and 7% Formula IV buprenorphine prodrug patch (n=4)**

| Compound | 72 h cumulative amt (nmol) | Flux (µg/cm²/h) | Lag time (h) |
|---|---|---|---|
| 5% Formula IV | 62.5 ± 17.5 | 0.9 ± 0.4 | 24.1 ± 11.2 |
| 7% Formula IV | 155.1 ± 48.5 | 1.5 ± 0.2 | 11.2 ± 5.8 |

The use of the terms "a" and "an" and "the" and similar references in the context of this disclosure (especially in the context of the following claims) are to be construed to cover both the singular and the plural, unless otherwise indicated herein or clearly contradicted by context. All methods and individual method steps described herein can be performed in any suitable order or simultaneously unless otherwise indicated herein or otherwise clearly contradicted by context. The use of any and all examples, or exemplary language (e.g., such as, preferred, preferably) provided herein, is intended merely to further illustrate the content of the disclosure and does not pose a limitation on the scope of the claims. No language in the specification should be construed as indicating any non-claimed element as essential to the practice of the present disclosure.

The use of individual numerical values are stated as approximations as though the values were preceded by the word "about" or "approximately." Similarly, the numerical values in the various ranges specified in this application, unless expressly indicated otherwise, are stated as approximations as though the minimum and maximum values within the stated ranges were both preceded by the word "about" or "approximately." In this manner, variations above and below the stated ranges can be used to achieve substantially the same results as values within the ranges. As used herein, the terms "about" and "approximately" when referring to a numerical value shall have their plain and ordinary meanings to a person of ordinary skill in the art to which the disclosed subject matter is most closely related or the art relevant to the range or element at issue. The amount of broadening from the strict numerical boundary depends upon many factors. For example, some of the factors which may be considered include the criticality of the element and/or the effect a given amount of variation will have on the performance of the claimed subject matter, as well as other considerations known to those of skill in the art. As used herein, the use of differing amounts of significant digits for different numerical values is not meant to limit how the use of the words "about" or "approximately" will serve to broaden a particular numerical value or range. Thus, as a general matter, "about" or "approximately" broaden the numerical value. Also, the disclosure of ranges is intended as a continuous range including every value between the minimum and maximum values plus the broadening of the range afforded by the use of the term "about" or "approximately." Thus, recitation of ranges of values herein are merely intended to serve as a shorthand method of referring individually to each separate value falling within the range, unless otherwise indicated herein, and each separate value is incorporated into the specification as if it were individually recited herein.

It is to be understood that any ranges, ratios and ranges of rations that can be formed by, or derived from, any of the data disclosed herein represents further embodiments of the present disclosure and are included as a part of the disclosure as though they were explicitly set forth. This includes ranges that can be formed that do or do not include a finite upper and/or lower boundary.

## Claims

1. A compound selected from the group consisting of: 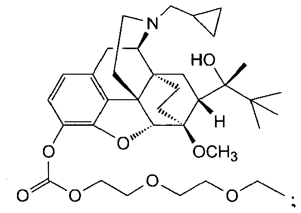 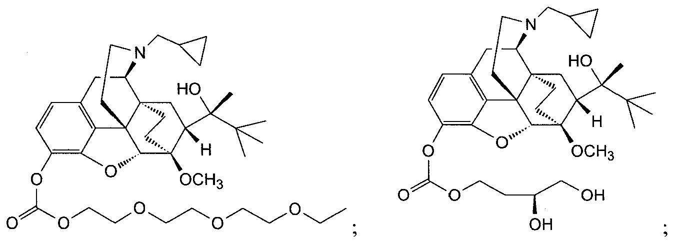 and salts of the foregoing.

2. A pharmaceutical composition comprising:
(a) a buprenorphine prodrug or a salt thereof of claim 1, and
(b) a pharmaceutical excipient.

3. The pharmaceutical composition of claim 2 further comprising a second compound selected from the group consisting of: naltrexone, prodrugs of naltrexone, naloxone and prodrugs of naloxone.

4. The pharmaceutical composition of claim 2, further comprising a second compound selected from the group consisting of: 3-O-pivalyl naltrexone; 3-O-isovaleryl naltrexone; 3-O-(2'-ethylbutyryl) naltrexone; 3-O-isobutyryl naltrexone; 3-O-isopropyloxycarbonyl naltrexone; 3-O-tertiarybutyloxycarbonyl naltrexone; N,N-dimethyl-3-O-carbamate naltrexone; N,N-diethyl-3-O-carbamate naltrexone; N,N-diisopropyl-3-O-carbamate naltrexone and a compound having the formula: 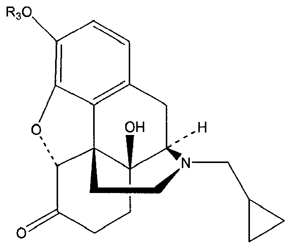 and salts thereof;
wherein R₃ is selected from the group consisting of: H, -COC(CH₃)₃, -COCH(CH₃)₂, -COCH_{2C}H(CH₃)₂, -COCH(CH₂CH₃)₂, -CON(CHCH₃)₂, -CON(CH(CH₃)₂)₂, -COOCH(CH₃)₂, 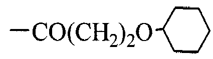 and -CO(CH₂)₂OCH₃.

5. A buprenorphine prodrug or salt thereof of claim 1 or a pharmaceutical composition of claim 2, 3 or 4 for use in the treatment of a medical condition selected from the group consisting of: opioid dependence, alcohol dependence, polydrug addiction, pain, cocaine addiction, eating disorders and treatment-resistant depression.

6. Use of the buprenorphine prodrug or salt thereof of claim 1 and a pharmaceutical acceptable excipient for the manufacture of a pharmaceutical composition, wherein the pharmaceutical composition is to be contacted with the skin of a mammal.

7. Use of the buprenorphine prodrug or salt thereof of claim 1 and a pharmaceutical acceptable excipient for the manufacture of a pharmaceutical composition according to claim 6 wherein the pharmaceutical composition further comprises a naltrexone prodrug of having the formula 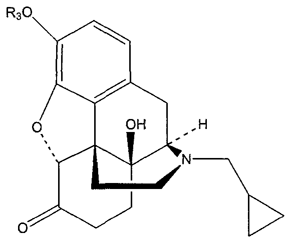 wherein R₃ is selected from the group consisting of: H; -COC(CH₃)₃; -COCH(CH₃)₂; -COCH₂CH(CH₃)₂; -COCH(CH₂CH₃)₂; -CON(CH₂CH₃)₂; -CON(CH(CH₃)₂;-COOCH(CH₃)₂; 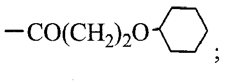 and -CO(CH₂)₂OCH₃.

8. Use of the buprenorphine prodrug or salt thereof of claim 1 and a pharmaceutical acceptable excipient for the manufacture of a pharmaceutical composition according to claim 6 wherein the pharmaceutical composition further comprises naloxone or a naloxone prodrug.

## Patentansprüche

1. Eine Verbindung ausgewählt aus der Gruppe bestehend aus: 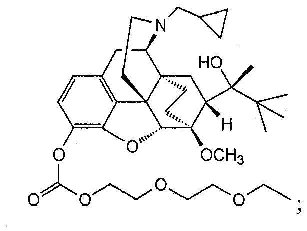 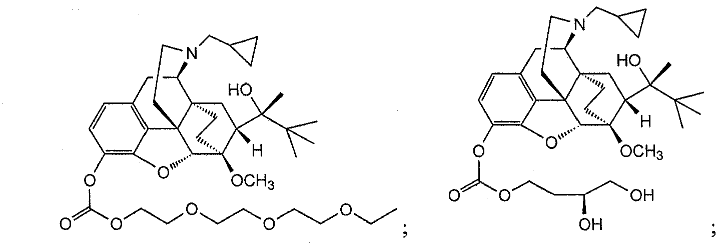 und Salze der vorhergehenden.

2. Eine pharmazeutische Zusammensetzung umfassend:
(a) ein Buprenorphin-Prodrug oder ein Salz davon nach Anspruch 1, und
(b) einen pharmazeutischen Hilfsstoff.

3. Die pharmazeutische Zusammensetzung nach Anspruch 2, weiter umfassend eine zweite Verbindung ausgewählt aus der Gruppe bestehend aus: Naltrexon, Naltrexon- Pro-Pharmaka, Naloxon und Naloxon-Pro-Pharmaka.

4. Die pharmazeutische Zusammensetzung nach Anspruch 2, weiter umfassend eine zweite Verbindung ausgewählt aus der Gruppe bestehend aus: 3-O-Pivalylnaltrexon; 3-O-Isovalerylnaltrexon; 3-O-(2'-Ethylbutyryl)naltrexon; 3-O-Isobutyrylnaltrexon; 3-O-Isopropyloxycarbonylnaltrexon; 3-O-*tert*-Butyloxycarbonylnaltrexon; N,N-Dimethyl-3-O-carbamatnaltrexon; N,N-Diethyl-3-O-Carbamatnaltrexon; N,N-Diisopropyl-3-O-carbamatnaltrexon und eine Verbindung mit der Formel: 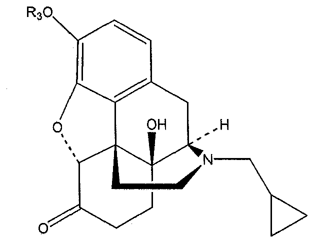 und Salze davon;
wobei R₃ ausgewählt ist aus der Gruppe bestehend aus: H, -COC(CH₃)₃, -COCH(CH₃)₂, -COCH₂CH(CH₃)₂, -COCH(CH₂CH₃)₂, -CON(CH₂CH₃)₂, -CON(CH(CH₃)₂)₂, -COOCH(CH₃)₂, 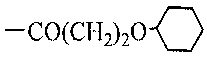 und -CO(CH₂)₂OCH₃.

5. Ein Buprenorphin-Prodrug oder ein Salz davon nach Anspruch 1 oder eine pharmazeutische Zusammensetzung nach Anspruch 2, 3 oder 4 zur Verwendung in der Behandlung einer Krankheit ausgewählt aus der Gruppe bestehend aus: Opioidabhängigkeit, Alkoholabhängigkeit, polyvalente Drogensucht, Schmerzen, Kokainsucht, Essstörungen und behandlungsresistente Depression.

6. Verwendung eines Buprenorphin-Prodrugs oder Salz davon nach Anspruch 1 und eines pharmazeutisch annehmbaren Hilfsstoffs zur Herstellung einer pharmazeutischen Zusammensetzung, wobei die pharmazeutische Zusammensetzung mit der Haut eines Säugetiers in Kontakt zu bringen ist.

7. Verwendung des Buprenorphin-Prodrugs oder Salz davon nach Anspruch 1 und eines pharmazeutisch annehmbaren Hilfsstoffs zur Herstellung einer pharmazeutischen Zusammensetzung entsprechend Anspruch 6,wobei die pharmazeutische Zusammensetzung weiter ein Naltrexon-Prodrug umfasst mit der Formel: 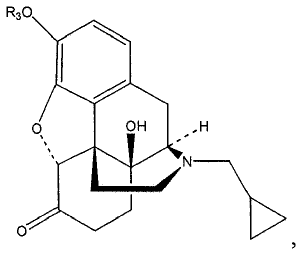 wobei R₃ ausgewählt ist aus der Gruppe bestehend aus: H; -COC(CH₃)₃; -COCH(CH₃)₂; -COCH₂CH(CH₃)₂; -COCH(CH₂CH₃)₂; -CON(CH₂CH₃)₂; -CON(CH(CH₃)₂)₂;-COOCH(CH₃)₂; 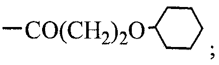 und -CO(CH₂)₂OCH₃.

8. Verwendung eines Buprenorphin-Prodrugs oder Salz davon nach Anspruch 1 und eines pharmazeutisch annehmbaren Hilfsstoffs zur Herstellung einer pharmazeutischen Zusammensetzung entsprechend Anspruch 6, wobei die pharmazeutische Zusammensetzung weiter ein Naloxon oder ein Naloxon-Prodrug umfasst.

## Revendications

1. Composé choisi parmi le groupe constitué de : 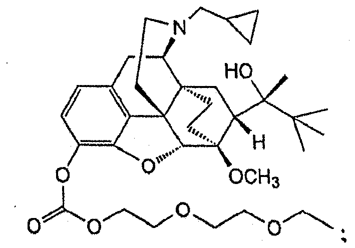 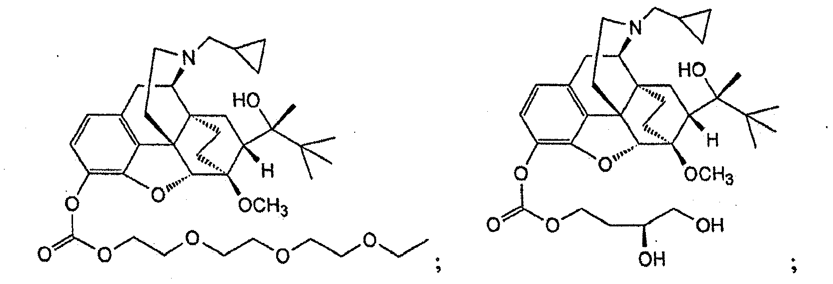 et des sels de ce qui précède.

2. Composition pharmaceutique comprenant :
(a) une prodrogue de buprénorphine ou un sel de celle-ci selon la revendication 1, et
(b) un excipient pharmaceutique.

3. Composition pharmaceutique selon la revendication 2 comprenant en outre un second composé choisi parmi le groupe constitué de : naltrexone, prodrogues de la naltrexone, naloxone et prodrogues de la naloxone.

4. Composition pharmaceutique selon la revendication 2, comprenant en outre un second composé choisi parmi le groupe constitué de : 3-O-pivalyl naltrexone ; 3-O-isovaléryl naltrexone ; 3-O-(2'-éthylbutyryl) naltrexone ; 3-O-isobutyryl naltrexone ; 3-O-isopropyloxycarbonyl naltrexone ; 3-O-tertiarybutyloxycarbonyl naltrexone ; N-N-diméthyl-3-O-carbamate naltrexone ; N,N-diéthyl-3-O-carbamate naltrexone ; N,N-diisopropyl-3-O-carbamate naltrexone et un composé de formule : 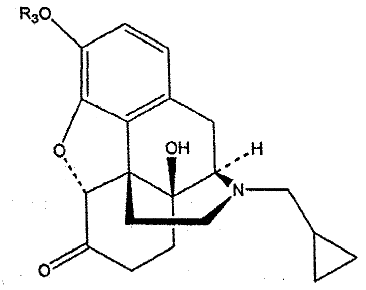 et les sels de celui-ci ; dans laquelle R₃ est choisi parmi le groupe constitué de : H, -COC(CH₃)₃, -COCH(CH₃)₂, - COCH₂CH(CH₃)₂, -COCH(CH₂CH₃)₂, -CON(CH₂CH₃)₂, - CON(CH(CH₃)₂)₂, -COOCH(CH₃)₂, 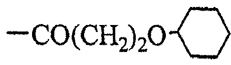 et - CO(CH₂)₂OCH₃.

5. Prodrogue de buprénorphine ou sel de celle-ci selon la revendication 1 ou composition pharmaceutique selon la revendication 2, 3 ou 4 pour son utilisation dans le traitement d'un état pathologique choisi parmi le groupe constitué de : la dépendance aux opioïdes, la dépendance à l'alcool, la polytoxicomanie, les douleurs, la dépendance à la cocaïne, les troubles de l'alimentation et la dépression résistante au traitement.

6. Utilisation d'une prodrogue de buprénorphine ou un sel de celle-ci selon la revendication 1 et un excipient pharmaceutique acceptable pour la préparation d'une composition pharmaceutique, dans laquelle la composition pharmaceutique doit être mise en contact avec la peau d'un mammifère.

7. Utilisation d'une prodrogue de buprénorphine ou un sel de celle-ci selon la revendication 1 et un excipient pharmaceutique acceptable pour la préparation d'une composition pharmaceutique selon la revendication 6, dans laquelle la composition pharmaceutique comprend en outre une prodrogue de naltrexone de formule 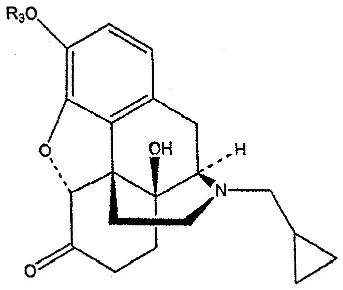 dans laquelle R₃ est choisi parmi le groupe constitué de : H, -COC(CH₃)₃, -COCH(CH₃)₂, - COCH₂CH(CH₃)₂, -COCH(CH₂CH₃)₂, -CON(CH₂CH₃)₂, - CON(CH(CH₃)₂)₂, -COOCH(CH₃)₂, 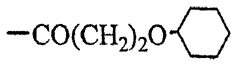 et-CO(CH₂)₂OCH₃.

8. Utilisation d'une prodrogue de buprénorphine ou un sel de celle-ci selon la revendication 1 et un excipient pharmaceutique acceptable pour la préparation d'une composition pharmaceutique selon la revendication 6, dans laquelle la composition pharmaceutique comprend en outre de la naloxone ou une prodrogue de naloxone.
